(19) Europäisches Patentamt
European Patent Office
Office européen des brevets

(11) **EP 4 170 577 A1**

(12) **EUROPEAN PATENT APPLICATION**
published in accordance with Art. 153(4) EPC

(43) Date of publication:
**26.04.2023 Bulletin 2023/17**

(21) Application number: **22807452.2**

(22) Date of filing: **09.05.2022**

(51) International Patent Classification (IPC):
**G06Q 50/10** (2012.01)     **G06Q 30/02** (2023.01)

(52) Cooperative Patent Classification (CPC):
**G06Q 30/02; G06Q 50/10**

(86) International application number:
**PCT/JP2022/019734**

(87) International publication number:
**WO 2022/239751 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.05.2021 JP 2021081158**

(71) Applicant: **Taste and Aroma Strategic Research Institute Co., Ltd.**
**Tokyo 104-0033 (JP)**

(72) Inventors:
• **KOYANAGI, Michihiro**
**Tokyo 104-0033 (JP)**
• **HAYASAKA, Hirofumi**
**Tokyo 104-0033 (JP)**
• **FUJIMARU, Junko**
**Tokyo 104-0033 (JP)**
• **KONDO, Tamaki**
**Tokyo 104-0033 (JP)**
• **TAKAHASHI, Takahiro**
**Tokyo 104-0033 (JP)**
• **MINEYOSHI, Yusuke**
**Tokyo 104-0033 (JP)**

(74) Representative: **Adamson Jones**
**Park View House**
**58 The Ropewalk**
**Nottingham NG1 5DW (GB)**

(54) **DEVICE FOR PRESENTING COMPATIBILITY INFORMATION ABOUT BEVERAGE AND FOOD, DISH AND SEASONING, SEASONINGS, AND MATERIAL COMPOSING EACH FOOD, AND METHOD FOR DIAGNOSING COMPATIBILITY BETWEEN BEVERAGE AND FOOD**

(57) To enable information on a combination having favorable compatibility of a drink and a food to be presented from a wide range as compared with the prior art. The item evaluation values of a drink and a food, of the relevant item and the relevant category as a pairing object, are extracted depending on a presentation request respectively from a drink item evaluation value storage section and a food item evaluation value storage section, and a drink and a food in a combination in which a correspondence relationship between the item evaluation values extracted of a drink and a food is matched to a correspondence relationship indicated by a compatibility pattern stored in a compatibility pattern storage section are determined to provide a combination having favorable compatibility. Information on a correspondence relationship between the item evaluation values of such drink and food determined to provide a combination having favorable compatibility is presented.

FIG.9

510

"SPECIFIC BRAND OF WHITE WINE" AND "GINGER PORK LOIN" PROVIDE COMBINATION HAVING FAVORABLE COMPATIBILITY

「ASTRINGENCY–RICHNESS」

DRINK     FOOD

「SOURNESS」          「DEEPNESS」

"SPECIFIC BRAND OF WHITE WINE" AND "GINGER PORK LOIN" EXHIBIT ITEM EVALUATION VALUES IV OF THREE EVALUATION ITEMS WELL–BALANCED AND "TUNED"

**Description**

Technical Field

The present invention relates to an apparatus capable of presenting information on a combination having favorable compatibility of a drink and a food, information on a combination having favorable compatibility of cuisine and a seasoning, information on a combination having favorable compatibility of seasonings, and information on a combination having favorable compatibility of food components, as well as a method of diagnosing compatibility of a drink and a food.

Background Art

(Conventional Art)

Patent Literature 1 (Japanese Patent Application Laid-Open (JP-A) No. 2017-130142) describes the invention relating to memorizing, as compatibility data, a combination having favorable compatibility of a kind of coffee and a kind of food in comparison among the respective degrees of intensities of sourness, bitterness, and saltiness.

SUMMARY OF INVENTION

Technical Problem

An object of the invention is to enable information on a combination having favorable compatibility of a drink and a food to be presented from a wide range as compared with the prior art.

Solution to Problem

A first aspect relates to a presentation apparatus of information on compatibility of a drink and a food, including a drink item evaluation value storage section in which an item evaluation value of each of a plurality of evaluation items for determination of compatibility with a food in terms of one or a combination of two or more of taste, odor, and eating quality, in association of a drink item and a food category as a pairing object, is stored as a relative numerical value to an item reference value, a food item evaluation value storage section in which an item evaluation value of each of the plurality of evaluation items for determination of compatibility with a drink in terms of one or a combination of two or more of taste, odor, and eating quality, in association of a food item and a drink category as a pairing object, is stored as a relative numerical value to an item reference value, a reception section which receives a presentation request of information on a combination having favorable compatibility of a drink and a food, a determination section which extracts item evaluation values of a drink and a food in the combination according to the presentation request received in the reception section, respectively from the drink item evaluation value storage section and the food item evaluation value storage section, and, in a case in which a correspondence relationship between the item evaluation values extracted of a drink and a food is matched to a specific correspondence relationship regarded as resulting in favorable compatibility, determines the drink and the food as a combination having favorable compatibility, and a presentation section which presents information on the drink and the food determined as the combination having favorable compatibility determined by the determination section.

A second aspect relates to the presentation apparatus of information on compatibility of a drink and a food of the first aspect, wherein the presentation section superposes and displays the item evaluation value of the drink and the item evaluation value of the food on a radar chart of the plurality of evaluation items.

A third aspect relates to the presentation apparatus of information on compatibility of a drink and a food of the first or second aspect, wherein the specific correspondence relationship regarded as resulting in favorable compatibility is prepared in the form of a plurality of compatibility patterns respectively exhibiting different correspondence relationships, and the presentation section presents information on a correspondence relationship between item evaluation values of a drink and a food determined as a combination having favorable compatibility, with respect to each of the plurality of compatibility patterns.

A fourth aspect relates to a presentation apparatus of information on compatibility of a drink and a food, including a drink item evaluation value storage section in which an item evaluation value of each of a plurality of evaluation items for determination of compatibility with a food in terms of one or a combination of two or more of taste, odor, and eating quality, in association of a drink item and a food category as a pairing object, is stored as a relative numerical value to an item reference value, a food item evaluation value storage section in which an item evaluation value of each of the plurality of evaluation items for determination of compatibility with a drink in terms of one or a combination of two or more of taste, odor, and eating quality, in association of a food item and a drink category as a pairing object, is stored as a relative numerical value to an item reference value, a drink-food combination item evaluation value storage section in which item evaluation values of a drink and a food in a specific combination in which a correspondence relationship between item evaluation values of a drink and a food stored in the drink item evaluation value storage section and the food item evaluation value storage section is matched to a specific correspondence relationship regarded as resulting in favorable compatibility are stored, a reception section which receives a presentation request of information on a combination having favorable compatibility of a drink and a food, and a presentation section which extracts item evaluation values of a drink and a food in the combination according to the presentation request received in the reception section, from the drink-food combination item evaluation value storage section, and presents information on the drink and the food extracted.

A fifth aspect relates to the presentation apparatus of information on compatibility of a drink and a food of the fourth aspect, wherein the presentation section superposes and displays the item evaluation value of the drink and the item evaluation value of the food on a radar chart of the plurality of evaluation items.

A sixth aspect relates to the presentation apparatus of information on compatibility of a drink and a food of the fourth or fifth aspect, wherein the specific correspondence relationship regarded as resulting in favorable compatibility is prepared in the form of a plurality of compatibility patterns respectively exhibiting different correspondence relationships, and the presentation section

presents information on a correspondence relationship between item evaluation values of a drink and a food determined as a combination having favorable compatibility, with respect to each of the plurality of compatibility patterns.

A seventh aspect relates to a presentation apparatus of information on compatibility of cuisine and a seasoning, including a cuisine item evaluation value storage section in which an item evaluation value of each of a plurality of evaluation items for determination of compatibility with a seasoning in terms of one or a combination of two or more of taste, odor, and eating quality, in association of a cuisine item and a seasoning category as a pairing object, is stored as a relative numerical value to an item reference value, a seasoning item evaluation value storage section in which an item evaluation value of each of the plurality of evaluation items for determination of compatibility with cuisine in terms of one or a combination of two or more of taste, odor, and eating quality, in association of a seasoning item and a cuisine category as a pairing object, is stored as a relative numerical value to an item reference value, a reception section which receives a presentation request of information on a combination having favorable compatibility of cuisine and a seasoning, a determination section which extracts item evaluation values of cuisine and a seasoning in the combination according to the presentation request received in the reception section, respectively from the cuisine item evaluation value storage section and the seasoning item evaluation value storage section, and, in a case in which a correspondence relationship between the item evaluation values extracted of cuisine and a seasoning is matched to a specific correspondence relationship regarded as resulting in favorable compatibility, determines the cuisine and the seasoning as a combination having favorable compatibility, and a presentation section which presents information on the cuisine and the seasoning determined as the combination having favorable compatibility determined by the determination section.

An eighth aspect relates to a presentation apparatus of information on compatibility of cuisine and a seasoning, including a cuisine item evaluation value storage section in which an item evaluation value of each of a plurality of evaluation items for determination of compatibility with a seasoning in terms of one or a combination of two or more of taste, odor, and eating quality, in association of a cuisine item and a seasoning category as a pairing object, is stored as a relative numerical value to an item reference value, a seasoning item evaluation value storage section in which an item evaluation value of each of the plurality of evaluation items for determination of compatibility with cuisine in terms of one or a combination of two or more of taste, odor, and eating quality, in association of a seasoning item and a cuisine category as a pairing object, is stored as a relative numerical value to an item reference value, a cuisine-seasoning combination item evaluation value storage section in which item evaluation values of cuisine and a seasoning in a specific combination in which a correspondence relationship between item evaluation values of cuisine and a seasoning stored in the cuisine item evaluation value storage section and the seasoning item evaluation value storage section is matched to a specific correspondence relationship regarded as resulting in favorable compatibility are stored, a reception section which receives a presentation request of information on a combination having favorable compatibility of cuisine and a seasoning, and a presentation section which extracts item evaluation values of cuisine and a seasoning in the combination according to the presentation request received in the reception section, from the cuisine-seasoning combination item evaluation value storage section, and presents information on the cuisine and the seasoning extracted.

A ninth aspect relates to a presentation apparatus of information on compatibility of seasonings, including a seasoning item evaluation value storage section in which an item evaluation value of each of the plurality of evaluation items for determination of compatibility with other seasoning in terms of one or a combination of two or more of taste, odor, and eating quality, in association of items of seasonings, is stored as a relative numerical value to an item reference value, a reception section which receives a presentation request of information on a combination having favorable compatibility of seasonings, a determination section which extracts each of item evaluation values of seasonings in the combination according to the presentation request received in the reception section, from the seasoning item evaluation value storage section, and, in a case in which a correspondence relationship between the item evaluation values extracted of seasonings is matched to a specific correspondence relationship regarded as resulting in favorable compatibility, determines the seasonings as a combination having favorable compatibility, and a presentation section which presents information on the seasonings as the combination having favorable compatibility determined by the determination section.

A tenth aspect relates to a presentation apparatus of information on compatibility of seasonings, including a seasoning item evaluation value storage section in which an item evaluation value of each of the plurality of evaluation items for determination of compatibility with other seasoning in terms of one or a combination of two or more of taste, odor, and eating quality, in association of items of seasonings, is stored as a relative numerical value to an item reference value, a seasoning combination item evaluation value storage section in which respective item evaluation values of seasonings in a specific combination in which a correspondence relationship between respective item evaluation values of seasonings stored in the seasoning item evaluation value storage section is a specific correspondence relationship regarded as resulting in favorable compatibility are stored, a reception section which receives a presentation request of information on a combination having favorable compatibility of seasonings, and a presentation section which extracts item evaluation values of seasonings in the combination according to the presentation request received in the reception section, from the seasoning combination item evaluation value storage section, and presents information on the seasonings extracted.

An eleventh aspect relates to a presentation apparatus of information on compatibility of food components, including a food component evaluation value storage section in which an evaluation value of each of a plurality of evaluation items for determination of compatibility with other food component in terms of one or a combination of two or more of taste, odor, and eating quality, in association of food components constituting a food, is stored as a relative numerical value to a food component reference value, a reception section which receives a presentation request of information on a combination having favorable compatibility of food components, a determination section which extracts each of evaluation values of food components in the combination according to the presentation request received in the reception section, from the food component evaluation value storage section, and, in a case in which a correspondence relationship between the evaluation values extracted of food components is matched to a specific correspondence relationship regarded as resulting in favorable compatibility, determines the food components as a combination having favorable compatibility, and a presentation section which presents information on the food components as the combination having favorable compatibility determined by the determination section.

A twelfth aspect relates to a presentation apparatus of information on compatibility of food components, including a food component evaluation value storage section in which an evaluation value of each of a plurality of evaluation items for determination of compatibility with other food component in terms of one or a combination of two or more of taste, odor, and eating quality, in association of food components, is stored as a relative numerical value to a food component reference value, a food component combination evaluation value storage section in which respective evaluation values of food components in a specific combination in which a correspondence relationship between respective evaluation values of food components stored in the food component evaluation value storage section is matched to a specific correspondence relationship regarded as resulting in favorable compatibility are stored, a reception section which receives a presentation request of information on a combination having favorable compatibility of food components, and a presentation section which extracts respective item evaluation values of food components in the combination according to the presentation request received in the reception section, from the food component combination item evaluation value storage section, and presents information on the food components extracted.

A thirteenth aspect relates to the presentation apparatus of information on compatibility of food components of the eleventh or twelfth aspect, wherein the plurality of evaluation items include hardness, viscosity and elasticity of a food component.

A fourteenth aspect relates to a method of diagnosing compatibility of a drink and a food, including diagnosing compatibility of a drink and a food by use of a detection value in a taste sensor, wherein the taste sensor is a sensor capable of measuring an evaluation value of aftertaste of a food, depending on a detection value obtained by immersion of a sensor section in a food sample liquid, then washing of the sensor section with a reference liquid corresponding to human saliva, and then immersion of the sensor section in the reference liquid, an evaluation value of aftertaste of a food due to flushing with a drink is measured depending on a detection value obtained by washing of the sensor section with a drink as a compatibility diagnosis target instead of washing of the sensor section with the reference liquid, and then immersion of the sensor section in the reference liquid, and compatibility of the drink and the food is diagnosed depending on the evaluation value of aftertaste of the food due to flushing with the drink.

A fifteenth aspect relates to the presentation apparatus of information on compatibility of a drink and a food of the first or fourth aspect, including a user-by-user history data storage section in which user-by-user history data showing a food item ever purchased by or supplied to a user, in association with the user, is stored, wherein, in a case in which the presentation request is received in the reception section, a food item is selected from the user-by-user history data stored in the user-by-user history data storage section, depending on the presentation request, and a drink item favorably compatible with the food item selected is displayed on a display screen of a user side terminal.

A sixteenth aspect relates to the presentation apparatus of information on compatibility of a drink and a food of the first or fourth aspect, including a user-by-user history data storage section in which user-by-user history data showing a drink item ever purchased by or supplied to a user, in association with the user, is stored, wherein, in a case in which the presentation request is received in the reception section, a drink item is selected from the user-by-user history data stored in the user-by-user history data storage section, depending on the presentation request, and a food item favorably compatible with the drink item selected is displayed on a display screen of a user side terminal.

A seventeenth aspect relates to the presentation apparatus of information on compatibility of cuisine and a seasoning of the seventh or eighth aspect, including a user-by-user history data storage section in which user-by-user history data showing a cuisine item ever purchased by or supplied to a user, in association with the user, is stored, wherein, in a case in which the presentation request is received in the reception section, a cuisine item is selected from the user-by-user history data stored in the user-by-user history data storage section, depending on the presentation request, and a seasoning item favorably compatible with the cuisine item selected is displayed on a display screen of a user side terminal.

An eighteenth aspect relates to the presentation apparatus of information on compatibility of cuisine and a seasoning of the seventh or eighth aspect, including a user-by-user history data storage section in which user-by-user history data showing a seasoning item ever purchased by or supplied to a user, in association with the user, is stored, wherein, in a case in which the presentation request is received in the reception section, a seasoning item is selected from the user-by-user history data stored in the user-by-user history data storage section, depending on the presentation request, and a cuisine item favorably compatible with the seasoning item selected is displayed on a display screen of a user side terminal.

A nineteenth aspect relates to the presentation apparatus of information on compatibility of seasonings of the ninth or tenth aspect, including a user-by-user history data storage section in which user-by-user history data showing a seasoning item ever purchased by or supplied to a user, in association with the user, is stored, wherein, in a case in which the presentation request is received in the reception section, a seasoning item is selected from the user-by-user history data stored in the user-by-user history data storage section, depending on the presentation request, and other seasoning item favorably compatible with the seasoning item selected is displayed on a display screen of a user side terminal.

A twentieth aspect relates to the presentation apparatus of information on compatibility of food components of the eleventh or twelfth aspect, including a user-by-user history data storage section in which user-by-user history data showing a food component ever purchased by or supplied to a user, in association with the user, is stored, wherein, in a case in which the presentation request is received in the reception section, a food component is selected from the user-by-user history data stored in the user-by-user history data storage section, depending on the presentation request, and other food component favorably compatible with the food component selected is displayed on a display screen of a user side terminal.

A twenty-first aspect relates to the presentation apparatus of information on compatibility of a drink and a food of the sixteenth aspect, wherein a food item providing a calorie intake in an acceptable range for the user is selected from the user-by-user history data.

A twenty-second aspect relates to the presentation apparatus of information on compatibility of cuisine and a seasoning of the eighteenth aspect, wherein a cuisine item providing a calorie intake in an acceptable range for the user is selected from the user-by-user history data.

A twenty-third aspect relates to the presentation apparatus of information on compatibility of food components of the twentieth aspect, wherein a food component item providing a calorie intake in an acceptable range for the user is selected from the user-by-user history data.

Advantageous Effect of Invention

According to the first to twenty-third aspects, any information on a combination having favorable compatibility of a drink and a food can be presented from a wide range as compared with the prior art.

BRIEF DESCRIPTION OF DRAWINGS

Figure 1 is a diagram illustrating a configuration of a presentation apparatus of information on compatibility of a drink and a food of an embodiment.
Figure 2 is a block diagram illustrating a hardware configuration of an embodiment.
Figure 3 is a block diagram illustrating a functional configuration of a presentation apparatus of information on compatibility of a drink and a food of a first embodiment.
Figure 4A is a diagram illustrating data of item evaluation values stored in a drink item evaluation value storage section and a food item evaluation value storage section.

Figure 4B is a diagram illustrating data of item evaluation values stored in a drink item evaluation value storage section and a food item evaluation value storage section.

Figure 4C is a diagram illustrating data of item evaluation values stored in a drink item evaluation value storage section and a food item evaluation value storage section.

Figure 5A is a diagram illustrating data stored in a drink item evaluation value storage section and a food item evaluation value storage section.

Figure 5B is a diagram illustrating data stored in a drink item evaluation value storage section and a food item evaluation value storage section.

Figure 6A is a diagram illustrating another data stored in a drink item evaluation value storage section and a food item evaluation value storage section.

Figure 6B is a diagram illustrating another data stored in a drink item evaluation value storage section and a food item evaluation value storage section.

Figure 7 illustrates first to fourth compatibility patterns stored in a compatibility pattern storage section.

Figure 8 illustrates an item evaluation value of "specific brand of white wine" illustrated in Figure 5A and an item evaluation value of "ginger pork loin" illustrated in Figure 5B, each superposed on a radar chart of each evaluation item of "astringency-richness", "sourness", and "deepness".

Figure 9 illustrates a drink-food compatibility information presentation screen displayed on a display apparatus of a user side terminal.

Figure 10 illustrates a drink-food compatibility information presentation screen displayed on a display apparatus of a user side terminal.

Figure 11 illustrates an item evaluation value of the item "specific brand of white wine" and an item evaluation value of the item "salad chicken", each superposed on a radar chart of each evaluation item of "astringency-richness", "sourness", and "deepness".

Figure 12 illustrates a drink-food compatibility information presentation screen displayed on a display apparatus of a user side terminal.

Figure 13A illustrates an item evaluation value of the item "specific brand of beer" and an item evaluation value of the item "Zha cai", each superposed on a radar chart of each evaluation item of "bitterness-richness", "sourness-sharpness", and "saltiness-strongness".

Figure 13B illustrates an item evaluation value of the item "specific brand of beer" and an item evaluation value of the item "boiled soybean", each superposed on a radar chart of each evaluation item of "bitterness-richness", "sourness-sharpness", and "saltiness-strongness" .

Figure 13C illustrates an item evaluation value of the item "specific brand of beer" and an item evaluation value of the item "char-broiled chicken", each superposed on a radar chart of each evaluation item of "bitterness-richness", "sourness-sharpness", and "saltiness-strongness".

Figure 14 is a block diagram illustrating a functional configuration of a presentation apparatus of information on compatibility of a drink and a food of a second embodiment.

Figure 15 illustrates a display screen displaying information on the item "roast fish (salt-grilled pacific saury)" and the item "citrus juice (citrus sudachi juice)" which are determined to be favorably compatible with each other.

Figure 16 illustrates a display screen displaying information on the item "deep-fried food" and the item "lemon juice" which are determined to be favorably compatible with each other.

Figure 17 illustrates a display screen displaying information on the item "broth soy sauce" and the item "citrus fruit" which are determined to be favorably compatible with each other.

Figure 18 illustrates a display screen displaying information on the item "soup stock (dried bonito/dried sea slug)" and the item "bean paste" which are determined to be favorably compatible with each other.

Figure 19 illustrates a display screen displaying information on the item "pork bone Chinese noodle soup" and the item "bean paste" which are determined to be favorably compatible with each other.

Figure 20A illustrates a combination pattern having favorable compatibility of food components.

Figure 20B illustrates a combination pattern having favorable compatibility of food components.

Figure 20C illustrates a combination pattern having favorable compatibility of food components.

Figure 21 illustrates a display screen displaying information on the item "fried egg" and the item "crispy bacon" which are determined to be favorably compatible with each other.

Figure 22 illustrates a display screen displaying information on the item "fermented soybean" and the item "okra" which are determined to be favorably compatible with each other.

Figure 23 is a graph with two evaluation axes of a horizontal axis representing the physical property value of "soft/hard" and a vertical axis representing the physical property value of "smooth/viscid".

Figure 24 is a table illustrating correspondence relationships between various volatile flavor components and odor qualities.

Figure 25 is a table representing the number of volatile compounds common between each alcoholic drink and a citrus iyo peel.

Figure 26 is a graph with two evaluation axes of a horizontal axis representing the evaluation value of "mild/gorgeous" and a vertical axis representing the evaluation value of "floral/fruity".

Figure 27 illustrates the evaluation value with respect to each odor evaluation item on a radar chart.

Figure 28A illustrates a procedure for measuring the aftertaste of a food with a taste sensor.

Figure 28B illustrates a procedure for measuring the aftertaste of a food due to flushing with a drink, with a taste sensor.

Figure 29A is a graph illustrating the numerical value of an evaluation value of the aftertaste of sushi (tuna) due to flushing with each of five brands of sakes.

Figure 29B illustrates the compatibility diagnosis results by use of the flushing aftertaste evaluation values.

Figure 30A is a graph illustrating the flushing aftertaste evaluation value in the case of a combination of pizza with each of sake, tea (green tea), and soft drink (coke).

Figure 30B is a graph illustrating the flushing aftertaste evaluation value in the case of a combination of pizza with each of sake, tea (green tea), and soft drink (coke).

Figure 31 illustrates a display screen displaying information on the food item "pizza (Margherita)" and the drink item "specific brand IT6 of sake" which are determined to be favorably compatible with each other.

Figure 32 illustrates a display screen displaying both a radar chart of evaluation items on six axes and a radar chart of evaluation items on three axes.

Figure 33 is a block diagram illustrating a functional configuration of a presentation apparatus of information on compatibility of Example 10.

Figure 34 is a block diagram illustrating a functional configuration of a presentation apparatus of information on compatibility of Example 10.

Figure 35A describes Example 10, and is a diagram illustrating a compatibility information presentation screen displayed on a display screen of a user side terminal.

Figure 35B describes Example 10, and is a diagram illustrating a compatibility information presentation screen displayed on a display screen of a user side terminal.

DESCRIPTION OF EMBODIMENTS

Hereinafter, embodiments of the invention will be described with reference to the drawings.

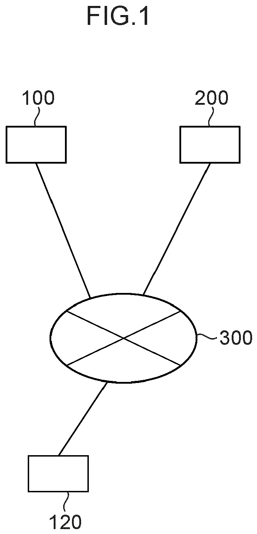

Figure 1 is a diagram illustrating a configuration of a presentation apparatus of information on compatibility of a drink and a food of an embodiment.

The presentation apparatus of information on compatibility of a drink and a food is configured from an administrator side terminal 100, a user side terminal 120, and a network 300 which connects each of the terminals 100 and 120 with a server 200 so that data can be sent and received mutually between each of the terminals 100 and 120 and the server 200.

The administrator side terminal 100 and the user side terminal 120 are each an information processing apparatus, and configured by, for example, a personal computer. The user side terminal 120 may be a mobile device such as a smartphone or a tablet. The network 300 is configured by, for example, internet and/or intranet. The server 200 is configured by, for example, server equipment or a virtual server constructed on cloud computing.

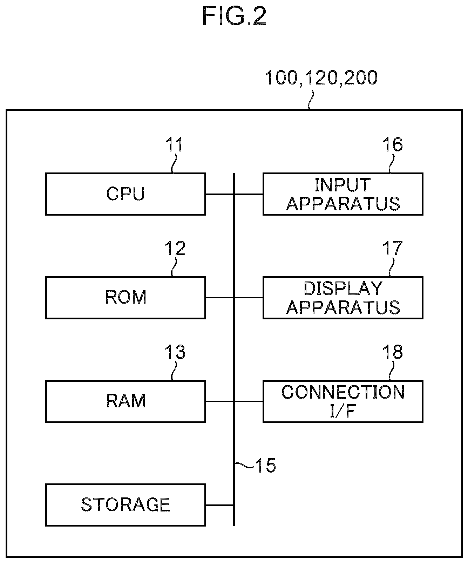

Figure 2 is a block diagram illustrating a hardware configuration of an administrator side terminal 100, a user side terminal 120 and a server 200 of an embodiment.

As illustrated in Figure 2, a CPU (Central Processing Unit) 11, a ROM (Read Only Memory) 12, a RAM (Random Access Memory) 13, a storage 14, an input apparatus 16, a display apparatus 17, and a connection I/F 18 are mutually and communicably connected via a system bus 15 in the administrator side terminal 100, the user side terminal 120, and the server 200.

The CPU 11 is a central processing unit, and executes various programs and/or controls each device connected to the system bus 15. In other words, the CPU 11 reads out a program from the ROM 12 or the storage 14 and executes the program with the RAM 13 as a workspace. The CPU 11 controls each device connected to the system bus 15 and performs various kinds of computing processing according to a program recorded in the ROM 12 or the storage 14. The ROM 12 or the storage 14 retains a BIOS (Basic Input/Output System) and an OS (Operating System) as control programs to be executed by the CPU 11, a computer-readable and executable program ("presentation program of information on compatibility of a drink and a food" for realization of the present embodiment, and various data required. The storage 14 is configured by an HDD (Hard Disk Drive) or an SSD (Solid State Drive).

The ROM 12 stores various kinds of control programs and various kinds of data. The RAM 13 functions as a main memory, a work area, or the like of the CPU 11, and functions as a workspace and temporarily memorizes any program or data.

The input apparatus 16 includes a pointing device such as a mouse, and a keyboard, and is used for performing various kinds of inputs.

The display apparatus 17 is, for example, a liquid crystal display, and displays various kinds of information. A touch panel system may be adopted in the display apparatus 17 and may serve as the input apparatus 16.

The display screen of the display apparatus 17 displays a drink-food compatibility information presentation screen 510 described below.

A communication interface 18 is an interface for communication with other equipment, and the standard of, for example, Ethernet (registered trademark), FDDI, or Wi-Fi (registered trademark) is used. The communication interface 18 is connected to the network 300 and controls sending and receiving of data.

The server 200 functions as a WEB server which allows a drink-food compatibility information presentation screen 510 to be displayed in the display apparatus 17 of the user side terminal 120 as a client terminal according to a communication protocol of HTTP (Hyper Text Transfer Protocol).

In a case in which the user side terminal 120 is accessed to the server 200, the drink-food compatibility information presentation screen 510 can be displayed in the display apparatus 17. The drink-food compatibility information presentation screen 510 presents (displays) information on a correspondence relationship between item evaluation values of a drink and a food which are favorably compatible with each other. The presentation herein used is meant to encompass not only any means with displaying, but also any output with a printer or the like.

The drink-food compatibility information presentation screen 510 may be here displayed on condition of user authentication.

(First Embodiment)

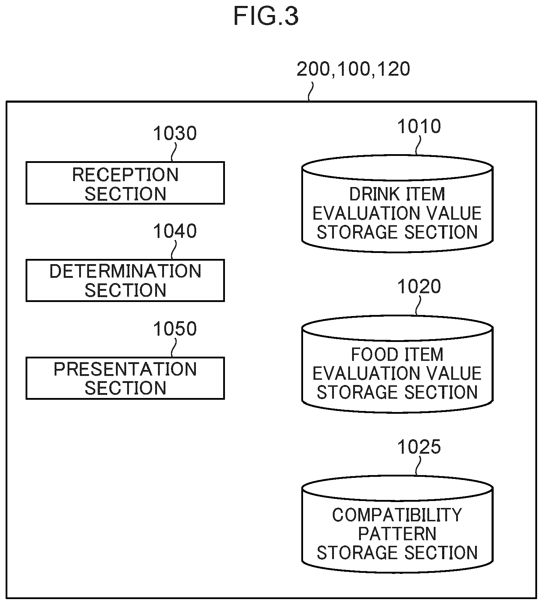

Figure 3 is a block diagram illustrating a functional configuration of a presentation apparatus of information on compatibility of a drink and a food of a first embodiment. The functional configuration illustrated in Figure 3 includes a server 200, an administrator side terminal 100, and a user side terminal 120.

The presentation apparatus of information on compatibility of a drink and a food is configured to include a drink item evaluation value storage section 1010, a food item evaluation value storage section 1020, a compatibility pattern storage section 1025, a reception section 1030, a determination section 1040, and a presentation section 1050. The drink item evaluation value storage section 1010, the food item evaluation value storage section 1020, and the compatibility pattern storage section 1025 are configured as databases, and are provided in the server 200. The drink item evaluation value storage section 1010, the food item evaluation value storage section 1020, and the compatibility pattern storage section 1025 may be configured as respective separate databases, or may be configured as an integral database.

(Drink Item Evaluation Value Storage Section, Food Item Evaluation Value Storage Section)

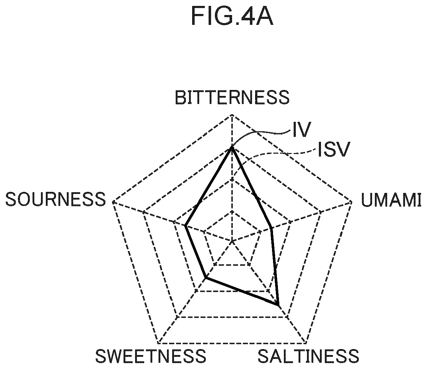

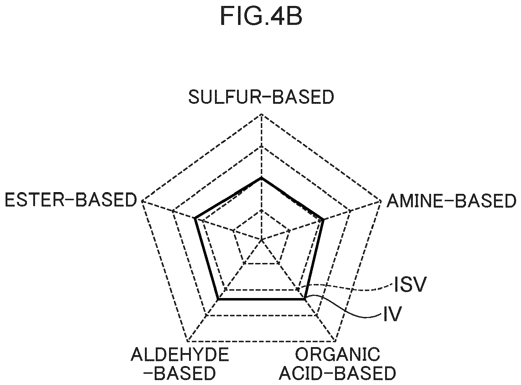

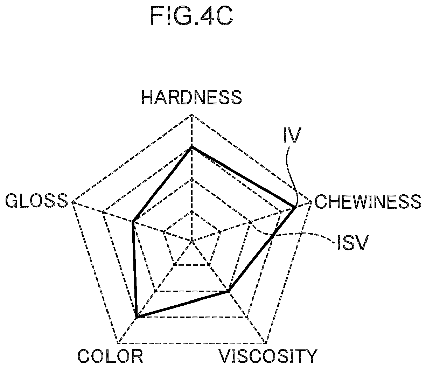

Figure 4A, Figure 4B, and Figure 4C each illustrate data of each item evaluation value IV stored in the drink item evaluation value storage section 1010 and the food item evaluation value storage section 1020.

As illustrated in Figure 4A, the drink item evaluation value storage section 1010 and the food item evaluation value storage section 1020 respectively store the item evaluation values IV with respect to each of a plurality of taste evaluation items (for example, sweetness, sourness, saltiness, bitterness, and umami) in association of a drink item and a food item.

As illustrated in Figure 4B, the drink item evaluation value storage section 1010 and the food item evaluation value storage section 1020 respectively store the item evaluation values IV with respect to each of a plurality of odor evaluation items (for example, sulfur-based, hydrocarbon-based, aromatic, ester-based, and aldehyde-based) in association of a drink item and a food item.

As illustrated in Figure 4C, the drink item evaluation value storage section 1010 and the food item evaluation value storage section 1020 respectively store the item evaluation values IV with respect to each of a plurality of eating quality evaluation items (for example, hardness, chewiness, viscosity, color, and gloss) in association of a drink item and a food item.

The "taste" refers to any sensation capable of being perceived by taste cells of the human tongue, and is intended to encompass respective evaluation items of basis five tastes including sweetness, sourness, saltiness, bitterness, and umami, respective evaluation items of broad tastes including pungency and astringency, and furthermore respective evaluation items indicated by sensibility, such as richness, sharpness, deepness, saltiness-strongness, afterglow of astringency, and body feeling.

The "odor" refers to fragrance or smell capable of being perceived by the human sense of smell, and is intended to encompass respective evaluation items indicated by chemical components such as ester smell and organic acid smell, and respective evaluation items indicated by sensibility, such as sweet fragrance, ginjo fragrance, and wood fragrance.

The "eating quality" refers to respective evaluation items such as texture, temperature, color, gloss, and sound, which can be perceived by the human senses of touch, vision, and hearing, and is intended to encompass respective evaluation items indicated by physical amounts such as viscosity, elasticity, and hardness, and respective evaluation items indicated by sensibility, such as chewiness and crunch feeling.

The drink item evaluation value storage section 1010 and the food item evaluation value storage section 1020 store each item evaluation value IV as a relative numerical value IDE (for example, +2.0) to an item reference value ISV (for example, 0.0).

The item evaluation value IV can be quantified based on a measurement value in a measurement apparatus. The value can also be quantified with a combination of the measurement value in a measurement apparatus and a sensory value obtained in sensory inspection. The measurement apparatus is intended to encompass a sensor stimulating a sensory organ perceiving the human senses of taste, smell, touch, vision, or hearing, and an analyzer for analysis of a component in a food or a drink.

The item evaluation value IV with respect to taste can be acquired based on a measurement value in a taste identification apparatus configured by, for example, a taste sensor and a component analyzer (gas chromatography, liquid chromatography, mass analyzer, infrared light absorption analyzer). The item evaluation value IV with respect to taste can also be acquired with a combination of a measurement value in a measurement apparatus and a sensory value obtained in sensory inspection.

The item evaluation value IV with respect to odor can be acquired based on a measurement value in an odor identification apparatus configured by, for example, an odor sensor and a component analyzer (gas chromatography or the like). The item evaluation value IV with respect to odor can also be acquired with a combination of a measurement value in a measurement apparatus and a sensory value obtained in sensory inspection.

The item evaluation value IV with respect to eating quality can be acquired based on a measurement value in an eating quality identification apparatus such as a visual analyzer (for measurement of, for example, color, gloss, shape, and/or pattern), a tensipresser (for measurement of, for example, chewing feeling and/or crunch feeling), a texturometer (for measurement of, for example, chewing feeling, hardness, or resilience), a rheometer (for measurement of, for example, dynamic viscoelasticity), a creep meter (for measurement of, for example, viscosity or elasticity), a viscometer (for measurement of, for example, viscosity), or a durometer (for measurement of, for example, hardness). The item evaluation value IV with respect to eating quality can also be acquired with a combination of a measurement value in a measurement apparatus and a sensory value obtained in sensory inspection.

For example, the taste sensor is configured so as to allow a plurality of lipid polymer membranes simulating biomembranes of taste cells of the tongue to receive a taste substance. Such a plurality of lipid polymers each respond to a taste substance of bitterness, sweetness, saltiness, sourness, umami, astringency, pungency, or the like, and then produce the potential difference with an electrode. The potential difference detected is converted into the item evaluation value IV with respect to bitterness, sweetness, saltiness, sourness, umami, astringency, pungency, or the like. The taste sensor allows for pseudo regeneration of the potential difference actually generated in sensory cells of the tongue by the taste substance itself, and thus the detection results in the taste sensor highly correlate to human senses.

The item reference value ISV refers to a numerical value serving as a reference of the item evaluation value IV, and an item evaluation value IV indicated by a reference sample RSA is desirably used. The item evaluation value IV here used can also be an average item evaluation value IV of each item in a category (for example, sake) to which an item (for example, specific brand of sake) belongs.

The reference sample RSA is defined with respect to each category of food-drink. The reference sample RSA is defined with respect to each category of food-drink, for example, sake, wine, beer, coffee, red tea, croquette, pork cutlet, and udon noodle soup. Such each category may correspond to a similar drink group or a similar food group. For example, the reference sample RSA serving as a reference for the item evaluation value IV of the item "brand A of red tea" or the item "brand B of oolong tea" may be adopted as the reference sample RSA of the category "tea group of red tea, oolong tea, green tea, and the like".

The reference sample RSA is configured by a standard composition which can allow an item reference value (for example, 0.0) to be certainly reproduced. Examples of the standard composition, in terms of sweetness, include glucose, fructose, and sucrose. Examples thereof, in terms of saltiness, include potassium chloride, potassium bromide, and sodium chloride. Examples thereof, in terms of sourness, include L(+)-tartaric acid, citric acid, and acetic acid. Examples thereof, in terms of umami, include sodium L-glutamate, sodium inosinate, sodium guanylate, and sodium succinate. Examples thereof, in terms of bitterness, include quinine hydrochloride, isoalpha acid, L-phenylalanine, and L-tryptophan. Examples thereof, in terms of astringency, include tannic acid, chlorogenic acid, coffeic acid, gallic acid, and epigallocatechin.

In an example of the category "sake", a liquid obtained by moderately mixing 2.0 g of glucose, 0.55 g of starch syrup, 0.20 g of thyrosol, 15 mL of ethanol, succinic acid, sodium succinate, sodium glutamate, glycine, alanine, sodium chloride, potassium dihydrogen phosphate, and/or the like can be used for 100 mL of the reference sample RSA in the sake category. A sample made with information on a standard composition of "2.0 g of glucose, 0.55 g of starch syrup, 15 mL of ethanol, and 0.20 g of thyrosol" can certainly realize an item reference value ISV serving as a reference of the item evaluation value IV of sake.

The reference sample RSA functions as a calibration sample.

A measurement apparatus, for example, the taste sensor, can highly cause an error. For example, in an example of the taste sensor, the detection value of potential difference in a certain period and the detection value of potential difference in another period may differ in numerical value, even in the case of a sample A in the same item. Thus, the item evaluation value IV is here determined depending on the relative value (for example, 5) of the detected potential difference (for example, 105) of the sample A, to the detected potential difference (for example, 100) of the reference sample RSA, detected in the same period. Thus, the same value (for example, 5) can be obtained as the relative value and any error of the item evaluation value IV can be avoided even in a case in which potential differences detected in different periods are each shifted by the same numerical value.

While the description is made with respect to taste, the same also applies to odor and eating quality.

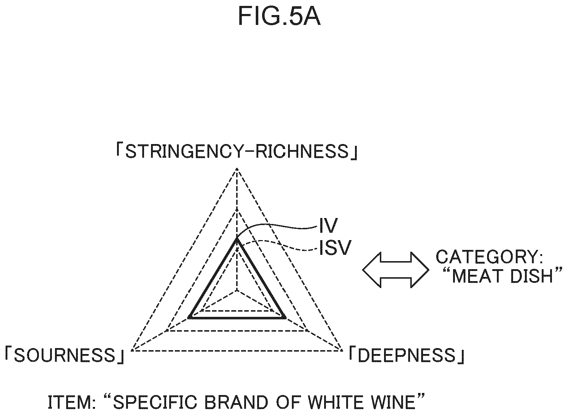

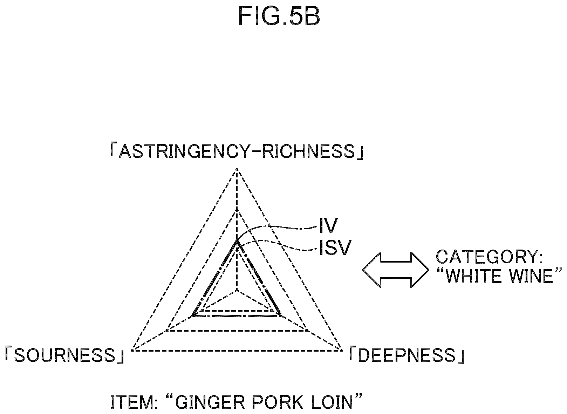

Figure 5A and Figure 5B respectively illustrate data stored in the drink item evaluation value storage section 1010 and the food item evaluation value storage section 1020.

Figure 5A represents, for example, the item evaluation value IV associated with the item "specific brand of white wine". The evaluation items "astringency-richness", "sourness", and "deepness" are selected in order to determine compatibility of the drink item "specific brand of white wine" and the food category "meat dish" as a pairing object.

Figure 5B represents, for example, the item evaluation value IV associated with the item "ginger pork loin". The evaluation items "astringency-richness", "sourness", and "deepness" are selected in order to determine compatibility of the item "ginger pork loin" and the drink category "white wine" as a pairing object.

Accordingly, in a case in which a correspondence relationship between the item evaluation value IV of the drink "specific brand of white wine" illustrated in Figure 5A and the item evaluation value IV of the food "ginger pork loin" illustrated in Figure 5B is matched to a compatibility pattern as a specific correspondence relationship regarded as resulting in favorable compatibility, both the drink and the food can be determined to be favorably compatible in the categories "white wine" and "meat dish".

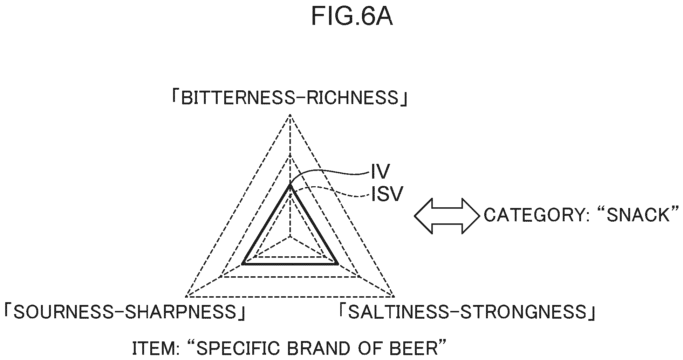

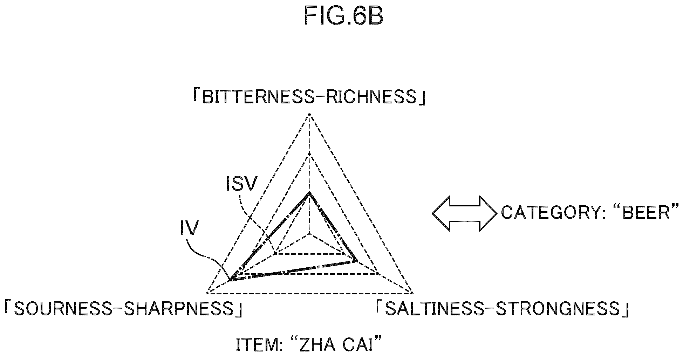

Figure 6A and Figure 6B respectively illustrate another data stored in the drink item evaluation value storage section 1010 and the food item evaluation value storage section 1020.

Figure 6A represents, for example, the item evaluation value IV associated with the item "specific brand of beer". The evaluation items "bitterness-richness", "sourness-sharpness", and "saltiness-strongness" are selected in order to determine compatibility of the drink item "specific brand of beer" and the food category "snack" as a pairing object.

Figure 6B represents, for example, the item evaluation value IV associated with the item "Zha cai". The evaluation items "bitterness-richness", "sourness-sharpness", and "saltiness-strongness" are selected in order to determine compatibility of the item "Zha cai" and the drink category "beer" as a pairing object.

Accordingly, in a case in which a correspondence relationship between the item evaluation value IV of the drink "specific brand of beer" illustrated in Figure 6A and the item evaluation value IV of the food "Zha cai" illustrated in Figure 6B is matched to a compatibility pattern as a specific correspondence relationship regarded as resulting in favorable compatibility, both the drink and the food can be determined to be favorably compatible in the categories "beer" and "snack".

The same also applies to other categories "sake" and "Japanese food", categories "green tea" and "confectionery", categories "coffee" and "dessert", and the like. The same data as the data illustrated in Figure 5 and Figure 6 is stored with respect to each of these categories in the drink item evaluation value storage section 1010 and the food item evaluation value storage section 1020. While the description is made with respect to taste, the data about odor and eating quality is also stored again in the drink item evaluation value storage section 1010 and the food item evaluation value storage section 1020.

(Compatibility Pattern Storage Section)

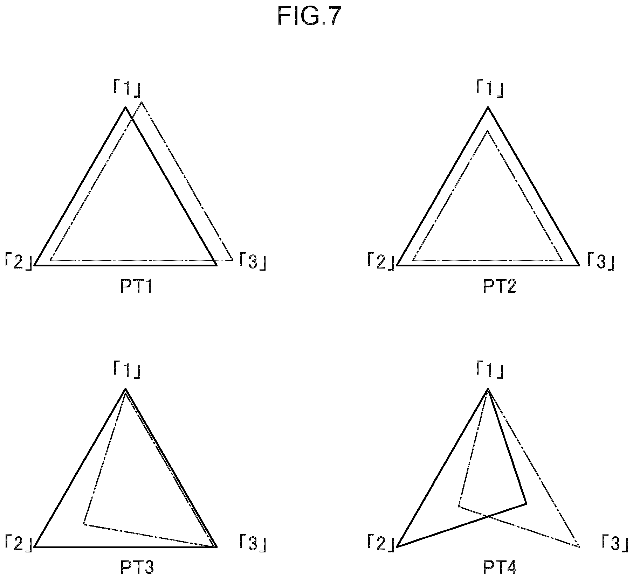

The compatibility pattern storage section 1025 stores first to fourth compatibility patterns PT1 to PT4 illustrated in Figure 7.

The first to fourth compatibility patterns PT1 to PT4 each indicate a specific correspondence relationship between the item evaluation values IV of a drink and a food considered to be favorably compatible with each other. The compatibility patterns PT1 to PT4 are respectively different correspondence relationships, as illustrated in Figure 7.

A case in which evaluation items for the item evaluation values IV are three evaluation items, for example, "astringency-richness", "sharpness", and "deepness" is described as an example. Figure 7 illustrates each line joining the item evaluation values IV of respective drink evaluation items "1", "2", "3", as a solid line, and each line joining the item evaluation values IV of respective food evaluation items "1", "2", "3", as a dash-dot-dash line.

· First Compatibility Pattern PT1

A correspondence relationship is meant in which matching or almost matching is obtained between a drink and a food in terms of all the item evaluation values IV of three evaluation items. Such drink and food can be determined to provide a combination having favorable compatibility in that taste intensities thereof are matched to each other, odor intensities thereof are matched to each other, and texture intensities thereof are matched to each other, and in that such a correspondence relationship is achieved with the item evaluation values IV of three evaluation items being balanced. The pattern is referred to as "tuned" compatibility pattern.

· Second Compatibility Pattern PT2

A correspondence relationship is meant in which approximation is obtained between a drink and a food in terms of all the item evaluation values IV of three evaluation items, although not as much as in the first compatibility pattern PT1. Such drink and food can be determined to provide a combination having favorable compatibility in that taste intensities thereof are approximate to each other, odor thereof are approximate to each other, and texture intensities thereof are matched to each other, and in that such a correspondence relationship is achieved with the item evaluation values IV of three evaluation items being balanced with approximation.

· Third Compatibility Pattern PT3

A correspondence relationship is meant in which matching or almost matching is obtained in terms of the item evaluation values IV of two evaluation items among the item evaluation values IV of three evaluation items and a large deviation is observed with respect to the item evaluation value IV of the remaining one evaluation item. A combination having favorable compatibility of a drink and a food can be determined in that such drink and food satisfy a correspondence relationship in which taste, odor, and texture not provided by one are complemented by those from other. Any savor is achieved in which the evaluation item (for example, "sharpness") with a large deviation observed stands out.

· Fourth Compatibility Pattern PT4

A correspondence relationship is meant in which matching or almost matching is obtained in terms of the item evaluation value IV of one evaluation item among the item evaluation values IV of three evaluation items and a large deviation is observed with respect to the item evaluation values IV of the remaining two evaluation items. The item evaluation value IV of one evaluation item (for example, "sharpness") in the remaining two evaluation items is larger in a drink, and the item evaluation value IV of another evaluation item (for example, "deepness") is larger in a food. A combination having favorable compatibility of a drink and a food can be determined in that such drink and food satisfy a correspondence relationship in which these are complemented with each other so that one provides any taste, odor, or texture not provided by other.

(Reception Section)

The "presentation program of information on compatibility of a drink and a food" is executed, whereby the processing of reception of the following presentation request, determination, and presentation of information on compatibility of a drink and a food is executed.
The reception section 1030 receives a presentation request of information on a combination having favorable compatibility of a drink and a food.
The presentation request may here specify any one item ("specific brand of white wine") of a drink and a food and request a favorably comparable item in a pairing object (for example, "meat dish") in other category. Alternatively, the presentation request may here specify both the categories of a drink and a food (for example, "white wine" and "meat dish") and request a combination item having favorable compatibility of a drink and a food in both the categories.
The presentation request is irrespective of the intension of a user. The presentation request may be made on the basis of the intension of a user, or may be recommended by another person, for example, a homepage administrator.
In an example of a presentation request of information on the "meat dish" going with the "specific brand of white wine", a presentation request is received and the presentation request of information on the "meat dish" going with the "specific brand of white wine" is presented to a user in a case in which the following events each occur.
A case in which a user specifies the drink item "specific brand of white wine" and also specifies the category "meat dish" as a pairing object on the screen of the display apparatus 17 of the user side terminal 120, and presses a button for request of information presentation
A case in which a user presses a button for presentation of recommended cuisine going with the "specific brand of white wine", on the screen of the display apparatus 17 of the user side terminal 120
· A case in which a user inputs a search term relating to the "specific brand of white wine", in a search box on the screen of the display apparatus 17 of the user side terminal 120

A case in which a user presses button for decision of buying of the "specific brand of white wine", on the screen of the display apparatus 17 of the user side terminal 120

The category as a pairing object may be here any of the "meat dish", "meat dish provided in specific store", "cuisine provided in specific store", or "cuisine (in general)".

(Determination Section)

The determination section 1040 extracts the item evaluation values IV of a drink (for example, "specific brand of white wine") and a food (for example, "meat dish") in the combination according to the presentation request received in the reception section 1030, respectively from the drink item evaluation value storage section 1010 and food item evaluation value storage section 1020, and, in a case in which a correspondence relationship between the item evaluation values extracted of a drink (for example, drink "specific brand of white wine") and a food (for example, "ginger pork loin") is matched to a specific correspondence relationship regarded as resulting in favorable compatibility (for example, first compatibility pattern PT1), determines the drink and the food as a combination having favorable compatibility.

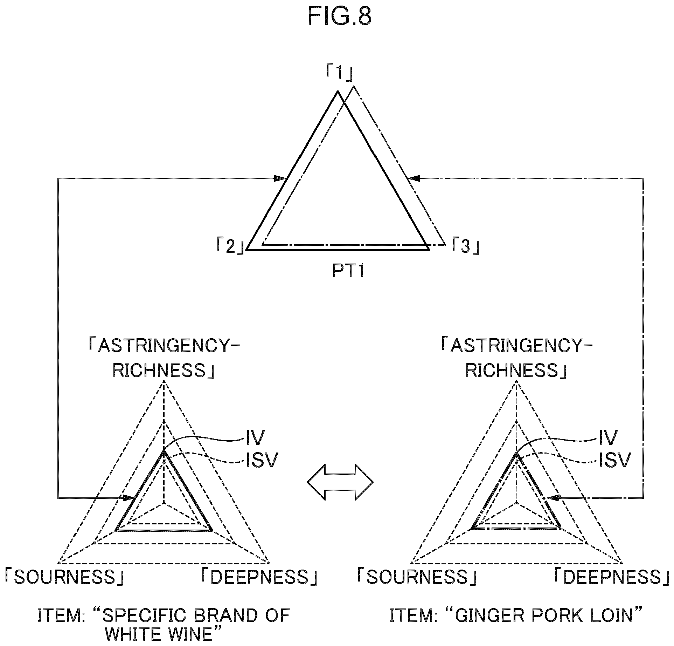

The content to be performed in the determination section 1040 is described with reference to Figure 5A, Figure 5B, and Figure 8. A case is assumed in which the presentation request of information on the "meat dish" going with the "specific brand of white wine" is made.

As illustrated in Figure 5A, the data of the item evaluation value IV with respect to each evaluation item of "astringency-richness", "sourness", and "deepness" of the item "specific brand of white wine" specified by the presentation request and the "specific brand of white wine" associated with the category "meat dish" as a pairing object is extracted from the drink item evaluation value storage section 1010. In this regard, as illustrated in Figure 5B, the data of the item evaluation value IV with respect to each evaluation item of "astringency-richness", "sourness", and "deepness" of the item "ginger pork loin" in the category "meat dish" as a pairing object, specified by the presentation request, and the "ginger pork loin" associated with the category "white wine" as a pairing object is extracted from the food item evaluation value storage section 1020.

Figure 8 illustrates processing for determining whether or not a correspondence relationship between the item evaluation value IV of the "specific brand of white wine" illustrated in Figure 5A and the item evaluation value IV of the "ginger pork loin" illustrated in Figure 5B is matched to the correspondence relationship indicated by the first compatibility pattern PT1.

The first compatibility pattern PT1 is extracted from the compatibility pattern storage section 1025. It is then determined whether or not the correspondence relationship between the item evaluation value IV of the "specific brand of white wine" illustrated in Figure 5A and the item evaluation value IV of the "ginger pork loin" illustrated in Figure 5B is matched to the specific correspondence relationship indicated by the first compatibility pattern PT1.

Matching or almost matching is obtained in terms of all the item evaluation values IV of three evaluation items "astringency-richness", "sourness", and "deepness" in the correspondence relationship between the item evaluation value IV of the "specific brand of white wine" illustrated in Figure 5A and the item evaluation value IV of the "ginger pork loin" illustrated in Figure 5B. Thus, it is determined that the correspondence relationship between the item evaluation value IV of the "specific brand of white wine" illustrated in Figure 5A and the item evaluation value IV of the "ginger pork loin" illustrated in Figure 5B is the specific correspondence relationship indicated by the first compatibility pattern PT1. Thus, the item "specific brand of white wine" and the item "ginger pork loin" are determined to provide a combination having favorable compatibility of a drink and a food in that the item evaluation values IV of three evaluation items are well-balanced and "tuned".

While the description is made with respect to taste, the same applies to odor and eating quality, and, as long as a correspondence relationship between the item evaluation value IV of a drink and the item evaluation value IV of a food is matched to any of the first to fourth compatibility patterns PT1 to PT4, such drink and food can be determined to provide a combination having favorable compatibility.

(Presentation Section)

The presentation section 1050 presents information on a drink and a food determined as a combination having favorable compatibility (for example, the item "specific brand of white wine" and the item "ginger pork loin"), depending on the determination result of the determination section 1040.

Hereinafter, each Example is described.

(Example 1)

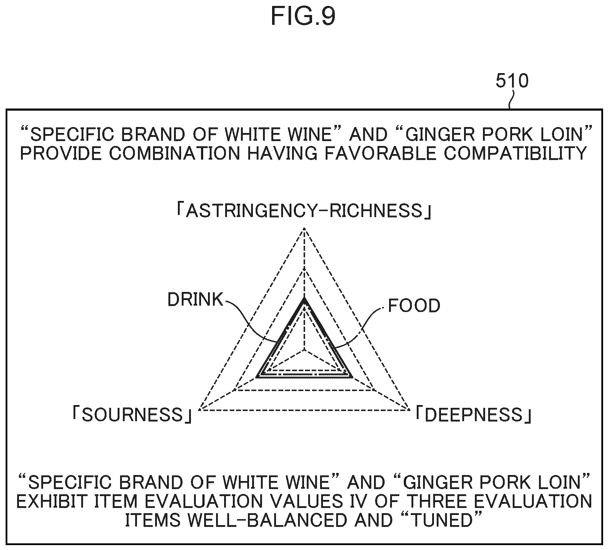

Figure 9 illustrates the drink-food compatibility information presentation screen 510 displayed on the display apparatus 17 of the user side terminal 120. The presentation section 1050 superposes and displays the item evaluation value IV of a drink and the item evaluation value IV of a food on the radar chart of each evaluation item of "astringency-richness", "sourness", and "deepness".

For example, in a case in which the presentation request of information on the "meat dish" going with the "specific brand of white wine" is made as described in Figure 8, the item "specific brand of white wine" and the item "ginger pork loin" are determined to provide a combination having favorable compatibility.

The display apparatus 17 of the user side terminal 120 not only displays information that the "specific brand of white wine" and the "ginger pork loin" provide a combination having favorable compatibility, depending on the determination result, but also superposes and displays the item evaluation value IV of the drink "specific brand of white wine" and the item evaluation value IV of the food "ginger pork loin" on the radar chart of each evaluation item of "astringency-richness", "sourness", and "deepness". The display apparatus may also display together information that the "specific brand of white wine" and the "ginger pork loin" exhibit the item evaluation values IV of three evaluation items well-balanced and "tuned".

Also in a case in which the food item "ginger pork loin" is specified and presentation of information on a favorably compatible drink item in the drink category "white wine" is requested, information that the "ginger pork loin" and the "specific brand of white wine" provide a combination having favorable compatibility can be displayed in the same manner as in Figure 9. The same also applies to a case in which both the drink and food categories "white wine" and "meat dish" are specified.

Thus, a user can find not only the trade name of a combination having favorable compatibility and the name of cuisine (the name of a drink product provided), but also the reason why such a combination is determined to be favorably compatible. For example, even a person who is not a specialist on paring can order a drink and/or a food (cuisine) in a restaurant, with confidence, based on the information indicated on the drink-food compatibility information presentation screen 510 displayed in the user side terminal 120 configured by a mobile device such as a smartphone or a tablet.

(Example 2)

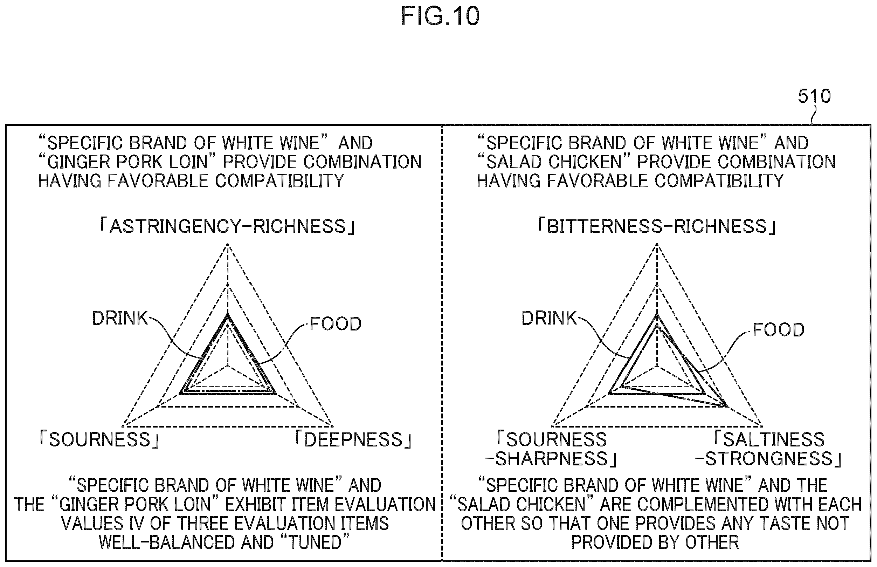

Figure 10 illustrates the drink-food compatibility information presentation screen 510 displayed on the display apparatus 17 of the user side terminal 120. The presentation section 1050 presents information on a drink and a food determined as a combination having favorable compatibility, with respect to each of a plurality of compatibility patterns, for example, the first compatibility pattern PT1 and the fourth compatibility pattern PT4, for example, information on the "specific brand of white wine" and the "ginger pork loin", and information on the "specific brand of white wine" and the "salad chicken".

For example, in a case in which the presentation request of information on the "meat dish" going with the "specific brand of white wine" is made as described in Figure 8, the item "ginger pork loin" in the category "meat dish" and the item "specific brand of white wine" are determined to provide a combination having favorable compatibility by application of the first compatibility pattern PT1.

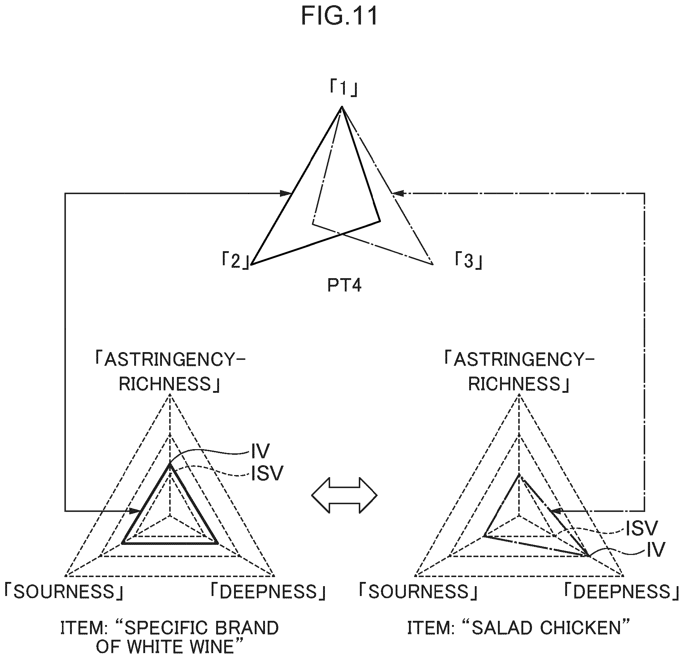

A case of application of the fourth compatibility pattern PT4 different from the first compatibility pattern PT1 is described with reference to Figure 11.

Figure 11 illustrates processing for determining whether or not a correspondence relationship between the item evaluation value IV of the item "specific brand of white wine" and the item evaluation value IV of the item "salad chicken" is matched to the correspondence relationship indicated by the fourth compatibility pattern PT4, in the same manner as in Figure 8.

The fourth compatibility pattern PT4 is extracted from the compatibility pattern storage section 1025. Whether or not the correspondence relationship between the item evaluation value IV of the item "specific brand of white wine" and the item evaluation value IV of the item "salad chicken", illustrated in Figure 11, is matched to the specific correspondence relationship indicated by the fourth compatibility pattern PT4 is determined.

The correspondence relationship between the item evaluation value IV of the item "specific brand of white wine" and the item evaluation value IV of the item "salad chicken" exhibits matching or almost matching in terms of the item evaluation value IV of one evaluation item "astringency-richness" in the item evaluation values IV of three evaluation items "astringency-richness", "sourness", and "deepness", and a large deviation in terms of the item evaluation values IV of the remaining two evaluation items "sharpness" and "deepness". Thus, the correspondence relationship between the item evaluation value IV of the item "specific brand of white wine" and the item evaluation value IV of the item "salad chicken" is determined to be the specific correspondence relationship indicated by the fourth compatibility pattern PT4. Thus, the item "specific brand of white wine" and the item "salad chicken" are determined to provide a combination having favorable compatibility of a drink and a food in that these satisfy a correspondence relationship in which these are complemented with each other so that one provides any taste not provided by other.

The display apparatus 17 of the user side terminal 120 displays information on the "specific brand of white wine" and the "salad chicken", in addition to information on the "specific brand of white wine" and the "ginger pork loin", depending on the determination results, as illustrated in Figure 10. The display apparatus not only displays information that the "specific brand of white wine" and the "salad chicken" provide a combination having favorable compatibility, but also superposes and displays the item evaluation value IV of the drink "specific brand of white wine" and the item evaluation value IV of the food "salad chicken" on the radar chart of each evaluation item of "astringency-richness", "sourness", and "deepness". The display apparatus may also display together information that the "specific brand of white wine" and the "salad chicken" are complemented with each other so that one provides any taste not provided by other.

(Example 3)

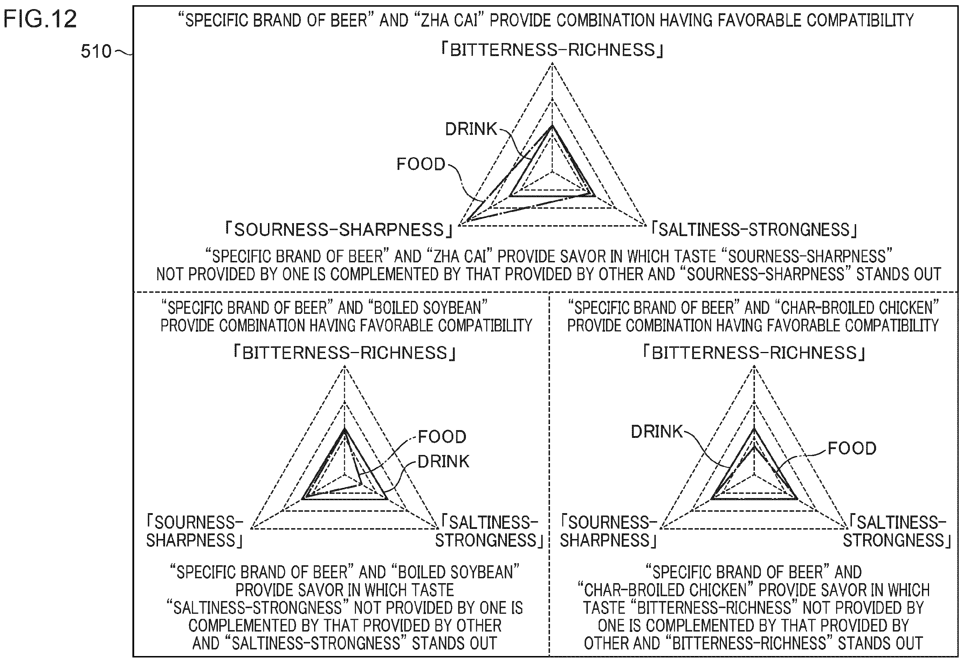

Figure 12 illustrates the drink-food compatibility information presentation screen 510 displayed on the display apparatus 17 of the user side terminal 120. The presentation section 1050 presents, for example, information on a plurality of combinations of drinks and foods which are favorably compatible with each other, with respect to the third compatibility pattern PT3, for example, information on three combinations of drinks and foods.

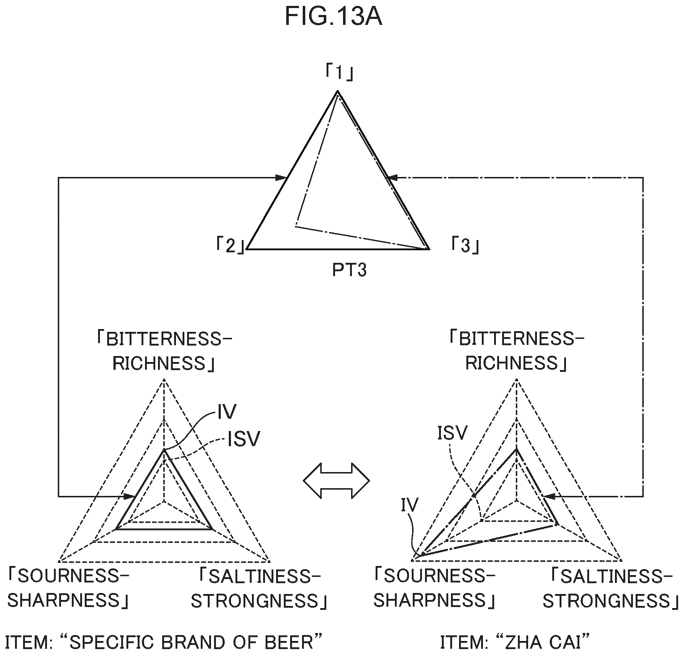

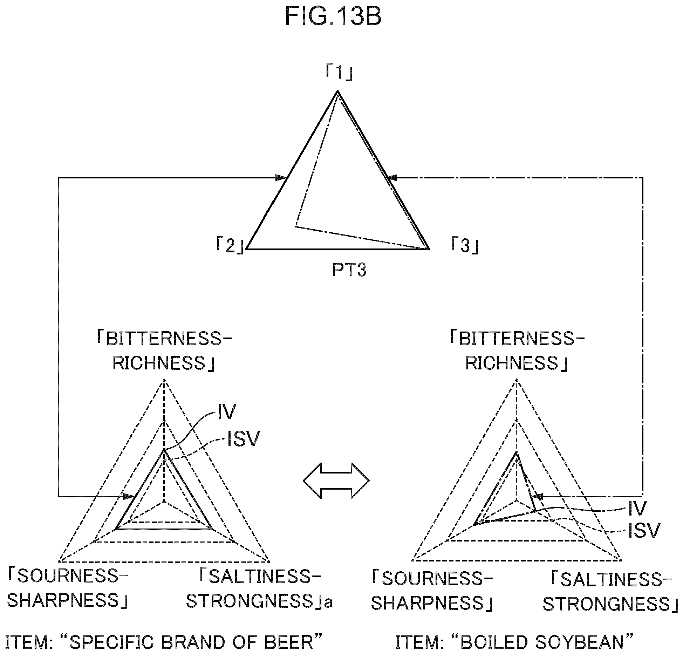

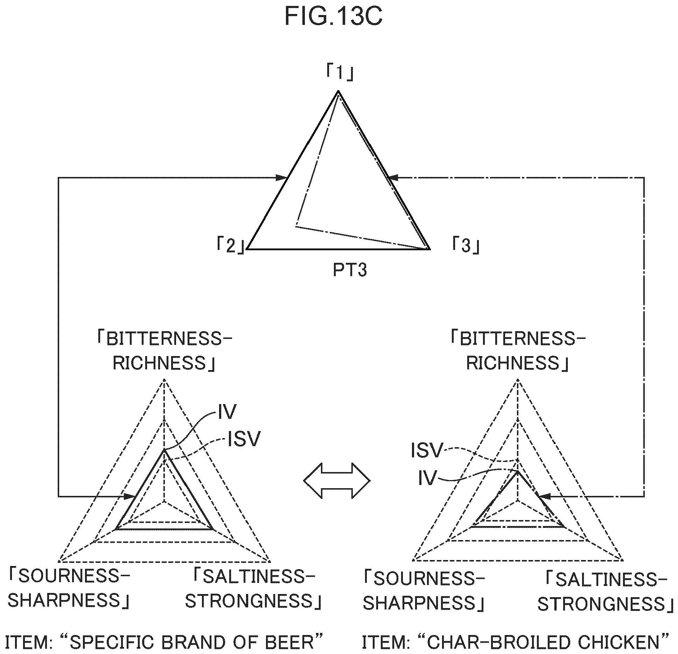

For example, a case in which a presentation request of information on the "snack" going with the "specific brand of beer" is made and the third compatibility pattern PT3 is applied is described with reference to Figure 13A, Figure 13B, and Figure 13C.

Figure 13A illustrates processing for determining whether or not a correspondence relationship between the item evaluation value IV of the item "specific brand of beer" illustrated in Figure 6A and the item evaluation value IV of the item "Zha cai" illustrated in Figure 6B is matched to the correspondence relationship indicated by the third compatibility pattern PT3.

The third compatibility pattern PT3 is extracted from the compatibility pattern storage section 1025. It is then determined whether or not the correspondence relationship between the item evaluation value IV of the item "specific brand of beer" illustrated in Figure 6A and the item evaluation value IV of the item "Zha cai" illustrated in Figure 6B is matched to the specific correspondence relationship indicated by the third compatibility pattern PT3.

The correspondence relationship between the item evaluation value IV of the item "specific brand of beer" and the item evaluation value IV of the item "Zha cai" exhibits matching or almost matching in terms of the item evaluation values IV of two evaluation items "bitterness-richness" and "saltiness-strongness" in the item evaluation values IV of three evaluation items "bitterness-richness", "sourness-sharpness", and "saltiness-strongness", and a large deviation in terms of the item evaluation value IV of the remaining one evaluation item "sourness-sharpness". The correspondence relationship between the item evaluation value IV of the item "specific brand of beer" and the item evaluation value IV of the item "Zha cai" is determined to be the specific correspondence relationship indicated by the third compatibility pattern PT3. Thus, the item "specific brand of beer" and the item "Zha cai" are determined to provide a combination having favorable compatibility of a drink and a food in that these satisfy a correspondence relationship of savor in which the taste "sourness-sharpness" not provided by one is complemented by that provided by other and the "sourness-sharpness" stands out.

Figure 13B illustrates processing for determining whether or not a correspondence relationship between the item evaluation value IV of the item "specific brand of beer" and the item evaluation value IV of the item "boiled soybean" is matched to the correspondence relationship indicated by the third compatibility pattern PT3, in the same manner as in Figure 13A.

The third compatibility pattern PT3 is extracted from the compatibility pattern storage section 1025. Whether or not the correspondence relationship between the item evaluation value IV of the item "specific brand of beer" and the item evaluation value IV of the item "boiled soybean" is matched to the specific correspondence relationship indicated by the third compatibility pattern PT3 is determined.

The correspondence relationship between the item evaluation value IV of the item "specific brand of beer" and the item evaluation value IV of the item "boiled soybean" exhibits matching or almost matching in terms of the item evaluation values IV of two evaluation items "bitterness-richness" and "sourness-sharpness" in the item evaluation values IV of three evaluation items "bitterness-richness", "sourness-sharpness", and "saltiness-strongness", and a large deviation in terms of the item evaluation value IV of the remaining one evaluation item "saltiness-strongness". Thus, the correspondence relationship between the item evaluation value IV of the item "specific brand of beer" and the item evaluation value IV of the item "boiled soybean" is determined to be the specific correspondence relationship indicated by the third compatibility pattern PT3. Thus, the item "specific brand of beer" and the item "boiled soybean" are determined to provide a combination having favorable compatibility of a drink and a food in that these satisfy a correspondence relationship of savor in which the taste "saltiness-strongness" not provided by one is complemented by that provided by other and the "saltiness-strongness" stands out.

Figure 13C illustrates processing for determining whether or not a correspondence relationship between the item evaluation value IV of the item "specific brand of beer" and the item evaluation value IV of the item "char-broiled chicken" is matched to the correspondence relationship indicated by the third compatibility pattern PT3, in the same manner as in Figure 13A and Figure 13B.

The third compatibility pattern PT3 is extracted from the compatibility pattern storage section 1025. Whether or not the correspondence relationship between the item evaluation value IV of the item "specific brand of beer" and the item evaluation value IV of the item "char-broiled chicken" is matched to the specific correspondence relationship indicated by the third compatibility pattern PT3 is determined.

The correspondence relationship between the item evaluation value IV of the item "specific brand of beer" and the item evaluation value IV of the item "char-broiled chicken" exhibits matching or almost matching in terms of the item evaluation values IV of two evaluation items "sourness-sharpness" and "saltiness-strongness" in the item evaluation values IV of three evaluation items "bitterness-richness", "sourness-sharpness", and "saltiness-strongness", and a large deviation in terms of the item evaluation value IV of the remaining one evaluation item "bitterness-richness". Thus, the correspondence relationship between the item evaluation value IV of the item "specific brand of beer" and the item evaluation value IV of the item "char-broiled chicken" is determined to be the specific correspondence relationship indicated by the third compatibility pattern PT3. Thus, the item "specific brand of beer" and the item "char-broiled chicken" are determined to provide a combination having favorable compatibility of a drink and a food in that these satisfy a correspondence relationship of savor in which the taste "bitterness-richness" not provided by one is complemented by that provided by other and the "bitterness-richness" stands out.

The display apparatus 17 of the user side terminal 120 displays information on the "specific brand of beer" and the "Zha cai", information on the "specific brand of beer" and the "boiled soybean", and information on the "specific brand of beer" and the "char-broiled chicken", depending on these determination results, as illustrated in Figure 12.

The information on the "specific brand of beer" and the "Zha cai", here displayed, is information that the "specific brand of beer" and the "Zha cai" provide a combination having favorable compatibility, and also the item evaluation value IV of the drink "specific brand of beer" and the item evaluation value IV of the food "Zha cai" are superposed and displayed on the radar chart of each evaluation item of "bitterness-richness", "sourness-sharpness", and "saltiness-strongness". In addition, information that the "specific brand of beer" and the "Zha cai" provide savor in which the taste "sourness-sharpness" not provided by one is complemented by that provided by other and the "sourness-sharpness" stands out may also be together displayed.

The information on the "specific brand of beer" and the "boiled soybean", here displayed, is information that the "specific brand of beer" and the "boiled soybean" provide a combination having favorable compatibility, and also the item evaluation value IV of the drink "specific brand of beer" and the item evaluation value IV of the food "boiled soybean" are superposed and displayed on the radar chart of each evaluation item of "bitterness-richness", "sourness-sharpness", and "saltiness-strongness". In addition, information that the "specific brand of beer" and the "boiled soybean" provide savor in which the taste "saltiness-strongness" not provided by one is complemented by that provided by other and the "saltiness-strongness" stands out may also be together displayed.

The information on the "specific brand of beer" and the "char-broiled chicken", here displayed, is information that the "specific brand of beer" and the "char-broiled chicken" provide a combination having favorable compatibility, and also the item evaluation value IV of the drink "specific brand of beer" and the item evaluation value IV of the food "char-broiled chicken" are superposed and displayed on the radar chart of each evaluation item of "bitterness-richness", "sourness-sharpness", and "saltiness-strongness". In addition, information on the "specific brand of beer" and the "char-broiled chicken" provide savor in which the taste "bitterness-richness" not provided by one is complemented by that provided by other and the "bitterness-richness" stands out may also be together displayed.

Next, a second embodiment is described.

(Second Embodiment)

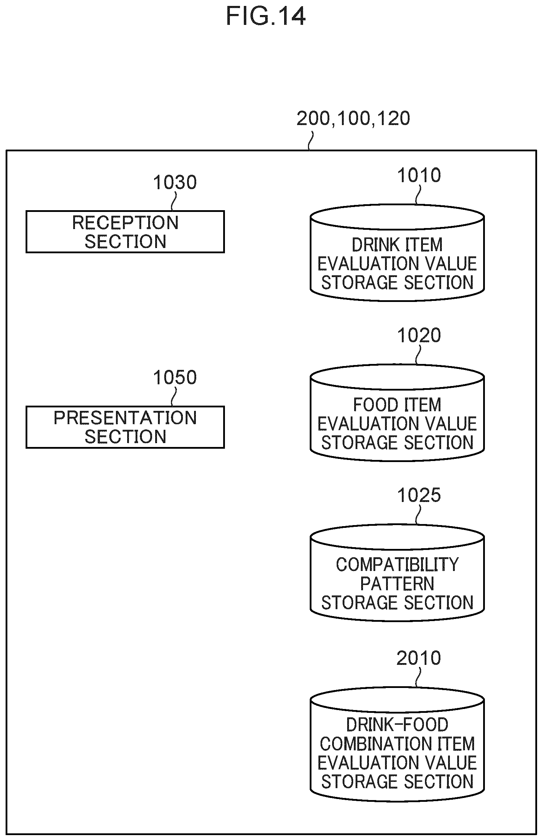

Figure 14 is a block diagram illustrating a functional configuration of a presentation apparatus of information on compatibility of a drink and a food of a second embodiment.

The same components as in the first embodiment are marked with the same symbols and reference numerals as in the first embodiment, and the descriptions thereof are appropriately omitted.

The functional configuration illustrated in Figure 14 includes a server 200, an administrator side terminal 100, and a user side terminal 120.

The presentation apparatus of information on compatibility of a drink and a food is configured to include a drink item evaluation value storage section 1010, a food item evaluation value storage section 1020, a compatibility pattern storage section 1025, a drink-food combination item evaluation value storage section 2010, a reception section 1030, and a presentation section 1050.

The drink item evaluation value storage section 1010, the food item evaluation value storage section 1020, the compatibility pattern storage section 1025, and the drink-food combination item evaluation value storage section 2010 are configured as databases, and are provided in the server 200. The drink item evaluation value storage section 1010, the food item evaluation value storage section 1020, the compatibility pattern storage section 1025, and the drink-food combination item evaluation value storage section 2010 may be configured as respective separate databases, or may be configured as an integral database.

The drink-food combination item evaluation value storage section 2010 stores the item evaluation values IV of a drink and a food in a specific combination in which a correspondence relationship between the item evaluation values IV of a drink and a food stored in the drink item evaluation value storage section 1010 and the food item evaluation value storage section 1020 is matched to a specific correspondence relationship regarded as resulting in favorable compatibility. The specific correspondence relationship is any of the first to fourth compatibility patterns PT1 to PT4 stored in the compatibility pattern storage section 1025.

Specifically, as illustrated in Figure 8, the drink-food combination item evaluation value storage section 2010 stores the data of the item evaluation values IV of a drink and a food in a specific combination in which the correspondence relationship between the item evaluation values IV of a drink and a food is matched to the specific correspondence relationship indicated by the first compatibility pattern PT1, regarded as resulting in favorable compatibility. As illustrated in Figure 11, the drink-food combination item evaluation value storage section 2010 stores the data of the item evaluation values IV of a drink and a food in a specific combination in which the correspondence relationship between the item evaluation values IV of a drink and a food is matched to the specific correspondence relationship indicated by the fourth compatibility pattern PT4, regarded as resulting in favorable compatibility. As illustrated in Figure 13A, Figure 13B, and Figure 13C, the drink-food combination item evaluation value storage section 2010 stores the data of the item evaluation values IV of a drink and a food in a specific combination in which the correspondence relationship between the item evaluation values IV of a drink and a food is matched to the specific correspondence relationship indicated by the third compatibility pattern PT3, regarded as resulting in favorable compatibility. Similarly, the drink-food combination item evaluation value storage section 2010 may store the data of the item evaluation values IV of a drink and a food in a specific combination in which the correspondence relationship between the item evaluation values IV of a drink and a food is matched to the specific correspondence relationship indicated by the second compatibility pattern PT2, regarded as resulting in favorable compatibility.

The reception section 1030 receives a presentation request of information on a combination having favorable compatibility of a drink and a food.

The presentation section 1050 allows for extraction of the item evaluation values IV of a drink and a food in the combination according to the presentation request received in the reception section 1030, from the drink-food combination item evaluation value storage section 2010, and presents the information on the drink and the food, extracted.

Specifically, as in the description of Example 1, in a case in which the presentation request of information on the "meat dish" going with the "specific brand of white wine" is made, the relevant data is extracted from the drink-food combination item evaluation value storage section 2010, and information on the item "specific brand of white wine" and the item "ginger pork loin" is presented on the display screen of the display apparatus 17 of the user side terminal 120, as illustrated in Figure 9.

As in the description of Example 2, in a case in which the presentation request of information on the "meat dish" going with the "specific brand of white wine" is made, the relevant data is extracted from the drink-food combination item evaluation value storage section 2010, and information on the "specific brand of white wine" and the "ginger pork loin" is displayed and also information on the "specific brand of white wine" and the "salad chicken" is presented on the display screen of the display apparatus 17 of the user side terminal 120, as illustrated in Figure 10.

As in the description of Example 3, in a case in which the presentation request of information on the "snack" going with the "specific brand of beer" is made, the relevant data is extracted from the drink-food combination item evaluation value storage section 2010, and information on the "specific brand of beer" and the "Zha cai", information on the "specific brand of beer" and the "boiled soybean", and information on the "specific brand of beer" and the "char-broiled chicken" are presented on the display screen of the display apparatus 17 of the user side terminal 120, as illustrated in Figure 12.

While the description is made above with respect to taste as an example, the same applies to odor and eating quality, and any similar information can be presented (displayed) on the display screen of the display apparatus 17 of the user side terminal 120.

In each Example below, a combination having favorable compatibility may be determined according to the presentation request and the determination result may be presented as in the first embodiment, or a combination having favorable compatibility may be stored in advance in a storage section and such a combination having favorable compatibility may be extracted from the storage section according to a provision request and then presented as in the second embodiment.

(Example 4)

The display screen 510 of the display apparatus 17 of the user side terminal 120 can similarly present (display) information on a combination having favorable compatibility of cuisine and a seasoning.

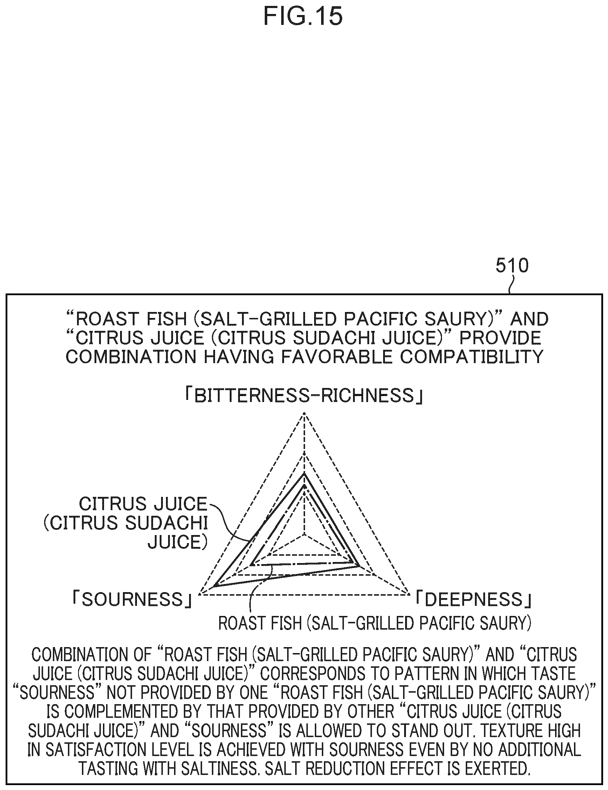

Figure 15 illustrates an example in which, in a case in which the presentation request of information on a seasoning going with cuisine is made, the display screen 510 of the display apparatus 17 of the user side terminal 120 presents information on the item "roast fish (salt-grilled pacific saury)" and the item "citrus juice (citrus sudachi juice)" determined to be favorably compatible with each other, as in the description of Example 1.

The item evaluation value IV of cuisine and the item evaluation value IV of seasoning are extracted from an evaluation value storage section not illustrated.

A correspondence relationship between the item evaluation value IV of the cuisine item "roast fish (salt-grilled pacific saury)" and the item evaluation value IV of the seasoning item "citrus juice (citrus sudachi juice)" exhibits matching or almost matching in terms of the item evaluation values IV of two evaluation items "bitterness-richness" and "deepness" in the item evaluation values IV of three evaluation items "bitterness-richness", "sourness", and "deepness", and a large deviation in terms of the item evaluation value IV of the remaining one evaluation item "sourness". The correspondence relationship between the item evaluation value IV of the item "roast fish (salt-grilled pacific saury)" and the item evaluation value IV of the item "citrus juice (citrus sudachi juice)" is determined to be the specific correspondence relationship indicated by the third compatibility pattern PT3. Thus, the item "roast fish (salt-grilled pacific saury)" and the item "citrus juice (citrus sudachi juice)" are determined to provide a combination having favorable compatibility of cuisine and a seasoning in that these satisfy a correspondence relationship in which the taste "sourness" not provided by one is complemented by that provided by other and the "sourness" is allowed to stand out.

The display screen 510 of the display apparatus 17 of the user side terminal 120 not only displays information that the "roast fish (salt-grilled pacific saury)" and the "citrus juice (citrus sudachi juice)" provide a combination having favorable compatibility, depending on the determination results, but also superposes and displays the item evaluation value IV of the cuisine "roast fish (salt-grilled pacific saury)" and the item evaluation value IV of the seasoning "citrus juice (citrus sudachi juice)" on the radar chart of each evaluation item of "bitterness-richness", "sourness", and "deepness", as illustrated in Figure 15. The display apparatus may also display together information that a combination of the "roast fish (salt-grilled pacific saury)" and the "citrus juice (citrus sudachi juice)" corresponds to a pattern in which the taste "sourness" not provided by one ("roast fish (salt-grilled pacific saury)") is complemented by that provided by other ("citrus juice (citrus sudachi juice)") and the "sourness" is allowed to stand out. The display apparatus may also display together information that "a texture high in satisfaction level is achieved with sourness even by no additional tasting with saltiness, and the salt reduction effect is exerted".

Even in a case in which seasonings are based on the same citrus juice, the items thereof may be subdivided into the "citrus sudachi juice", the "citrus yuzu ponzu sauce" .... Information on each citrus juice item favorably compatible with the "roast fish (pacific saury)" may be presented by arranging the "citrus sudachi juice", "citrus yuzu ponzu sauce", and the like, for example, in descending order of compatibility.

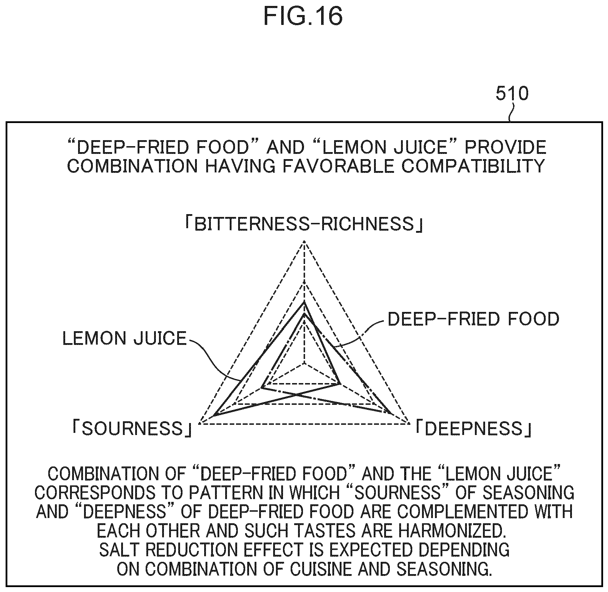

Figure 16 illustrates an example in which, in a case in which the presentation request of information on a seasoning going with cuisine is made, the display screen 510 of the display apparatus 17 of the user side terminal 120 presents information on the item "deep-fried food" and the item "lemon juice" determined to be favorably compatible with each other.

The item evaluation value IV of cuisine and the item evaluation value IV of a seasoning are extracted from an evaluation value storage section not illustrated.

A correspondence relationship between the item evaluation value IV of the cuisine item "deep-fried food" and the item evaluation value IV of the seasoning item "lemon juice" exhibits matching or almost matching in terms of the item evaluation value IV of one evaluation item "bitterness-richness" in the item evaluation values IV of three evaluation items "bitterness-richness", "sourness", and "deepness", and a large deviation in terms of the item evaluation values IV of the remaining two evaluation items "sourness" and "deepness". Thus, the correspondence relationship between the item evaluation value IV of the item "deep-fried food" and the item evaluation value IV of the item "lemon juice" is determined to be the specific correspondence relationship indicated by the fourth compatibility pattern PT4. Thus, the item "deep-fried food" and the item "lemon juice" are determined to provide a combination having favorable compatibility of cuisine and a seasoning in that these satisfy a correspondence relationship in which these are complemented with each other so that one provides any taste not provided by other.

The display screen 510 of the display apparatus 17 of the user side terminal 120 not only displays information that the "deep-fried food" and the "lemon juice" provide a combination having favorable compatibility, depending on the determination results, but also superposes and displays the item evaluation value IV of the cuisine "deep-fried food" and the item evaluation value IV of the seasoning "lemon juice" on the radar chart of each evaluation item of "bitterness-richness", "sourness", and "deepness", as illustrated in Figure 16. The display screen may also display together information that the combination of the "deep-fried food" and the "lemon juice" corresponds to a pattern in which "sourness" of such a seasoning and "deepness" of such a deep-fried food are complemented with each other and such tastes are harmonized. The display screen may also display together information that the salt reduction effect is expected depending on the combination of such cuisine and seasoning.

Any other "chopped onion dressing", besides the "lemon juice", may be present as the seasoning favorably compatible with the "deep-fried food". Information on each item of the seasoning favorably compatible with the "deep-fried food" may be presented by arranging the "lemon juice", "chopped onion dressing vinegar", and the like, for example, in descending order of compatibility.

(Example 5)

The display screen 510 of the display apparatus 17 of the user side terminal 120 also presents (displays) similarly information on a combination of seasonings favorably compatible with each other.

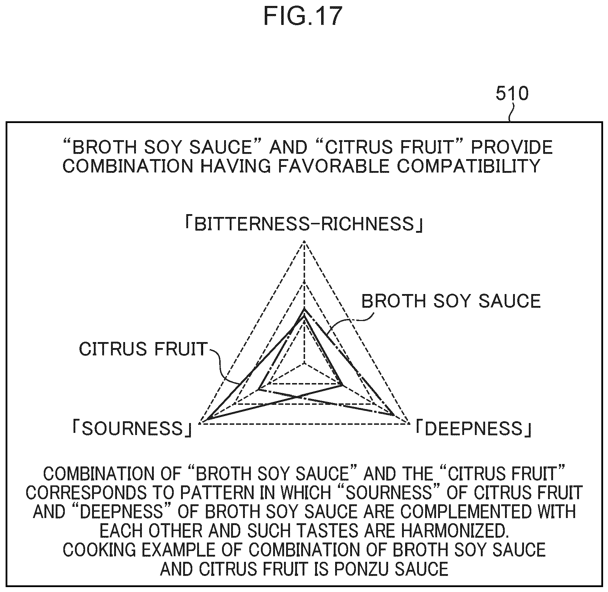

Figure 17 illustrates an example in which, in a case in which the presentation request of information on other seasoning going with a seasoning is made, the display screen of the display apparatus 17 of the user side terminal 120 presents information on the item "broth soy sauce" and the item "citrus fruit" determined to be favorably compatible with each other.

The item evaluation value IV of a seasoning is extracted from an evaluation value storage section not illustrated.

A correspondence relationship between the item evaluation value IV of the seasoning item "broth soy sauce" and the item evaluation value IV of the seasoning item "citrus fruit" exhibits matching or almost matching in terms of the item evaluation value IV of one evaluation item "bitterness-richness" in the item evaluation values IV of three evaluation items "bitterness-richness", "sourness", and "deepness", and a large deviation in terms of the item evaluation values IV of the remaining two evaluation items "sourness" and "deepness". Thus, the correspondence relationship between the item evaluation value IV of the item "broth soy sauce" and the item evaluation value IV of the item "citrus fruit" is determined to be the specific correspondence relationship indicated by the fourth compatibility pattern PT4. Thus, the item "broth soy sauce" and the item "citrus fruit" are determined to provide a combination having favorable compatibility of seasonings in that these satisfy a correspondence relationship in which these are complemented with each other so that one provides any taste not provided by other.

The display screen 510 of the display apparatus 17 of the user side terminal 120 displays not only information that the "broth soy sauce" and the "citrus fruit" provide a combination having favorable compatibility, depending on the determination results, but also superposes and displays the item evaluation value IV of the seasoning "broth soy sauce" and the item evaluation value IV of the seasoning "citrus fruit" on the radar chart of each evaluation item of "bitterness-richness", "sourness", and "deepness", as illustrated in Figure 17. The display screen may also display together information that the combination of the "broth soy sauce" and the "citrus fruit" corresponds to a pattern in which "sourness" of the citrus fruit and "deepness" of the broth soy sauce are complemented with each other and such tastes are harmonized. The display screen may also display together information that a cooking example of the combination of the broth soy sauce and the citrus fruit is a ponzu sauce.

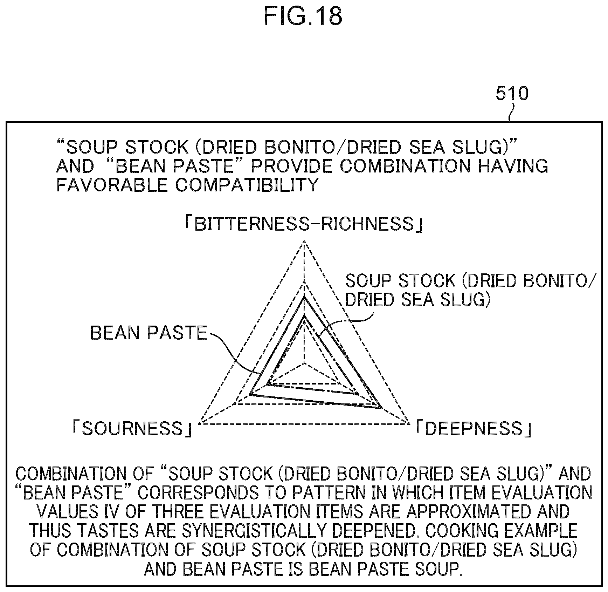

Figure 18 an example in which, in a case in which the presentation request of information on other seasoning going with a seasoning is made, the display screen 510 of the display apparatus 17 of the user side terminal 120 presents information on the item "soup stock (dried bonito/dried sea slug)" and the item "bean paste" determined to be favorably compatible with each other.

The item evaluation value IV of a seasoning is extracted from an evaluation value storage section not illustrated.

A correspondence relationship between the item evaluation value IV of the seasoning item "soup stock (dried bonito/dried sea slug)" and the item evaluation value IV of the seasoning item "bean paste" exhibits approximation at the same levels in terms of all the item evaluation values IV of three evaluation items "bitterness-richness", "sourness", and "deepness". Thus, the correspondence relationship between the item evaluation value IV of the item "soup stock (dried bonito/dried sea slug)" and the item evaluation value IV of the item "bean paste" is determined to be the specific correspondence relationship indicated by the second compatibility pattern PT2. Thus, the item "soup stock (dried bonito/dried sea slug)" and the item "bean paste" can be determined to provide a combination having favorable compatibility of seasonings in that a correspondence relationship is achieved in which the item evaluation values IV are balanced with approximation.

The display screen 510 of the display apparatus 17 of the user side terminal 120 not only displays information that the "soup stock (dried bonito/dried sea slug)" and the "bean paste" provide a combination having favorable compatibility, depending on the determination results, but also superposes and displays the item evaluation value IV of the cuisine "soup stock (dried bonito/dried sea slug)" and the item evaluation value IV of the seasoning "bean paste" on the radar chart of each evaluation item of "bitterness-richness", "sourness", and "deepness", as illustrated in Figure 18. The display screen may also display together information that the combination of the "soup stock (dried bonito/dried sea slug)" and the "bean paste" corresponds to a pattern in which the item evaluation values IV of three evaluation items of such cuisine and seasoning are approximated and thus tastes are synergistically deepened. The display screen may also display together information that a cooking example of the combination of the soup stock (dried bonito/dried sea slug) and the bean paste is a bean paste soup.

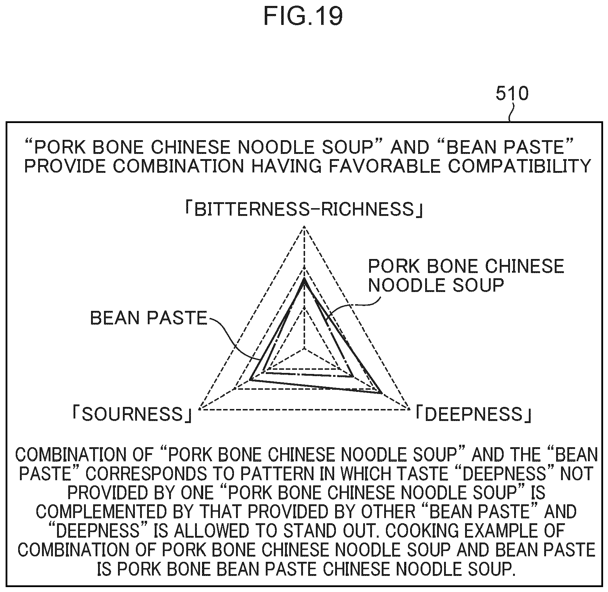

Figure 19 illustrates an example in which, in a case in which the presentation request of information on other seasoning going with a seasoning is made, the display screen 510 of the display apparatus 17 of the user side terminal 120 presents information on the item "pork bone Chinese noodle soup" and the item "bean paste" determined to be favorably compatible with each other.

The item evaluation value IV of a seasoning is extracted from an evaluation value storage section not illustrated.

A correspondence relationship between the item evaluation value IV of the seasoning item "pork bone Chinese noodle soup" and the item evaluation value IV of the seasoning item "bean paste" exhibits matching or almost matching in terms of the item evaluation values IV of two evaluation items "bitterness-richness" and "sourness" in the item evaluation values IV of three evaluation items "bitterness-richness", "sourness", and "deepness", and a large deviation in terms of the item evaluation value IV of the remaining one evaluation item "deepness". Thus, the correspondence relationship between the item evaluation value IV of the item "pork bone Chinese noodle soup" and the item evaluation value IV of the item "bean paste" is determined to be the specific correspondence relationship indicated by the third compatibility pattern PT3. Thus, the item "pork bone Chinese noodle soup" and the item "bean paste" can be determined to provide a combination having favorable compatibility of seasonings in that these satisfy a correspondence relationship in which the taste "deepness" not provided by one is complemented by that provided by other and the "deepness" is allowed to stand out.

The display screen 510 of the display apparatus 17 of the user side terminal 120 not only displays information that the "pork bone Chinese noodle soup" and the "bean paste" provide a combination having favorable compatibility, depending on the determination results, but also superposes and displays the item evaluation value IV of the cuisine "pork bone Chinese noodle soup" and the item evaluation value IV of the seasoning "bean paste" on the radar chart of each evaluation item of "bitterness-richness", "sourness", and "deepness", as illustrated in Figure 19. The display screen may also display together information that the combination of the "pork bone Chinese noodle soup" and the "bean paste" corresponds to a pattern in which the taste "deepness" not provided by one ("pork bone Chinese noodle soup") is complemented by that provided by other ("bean paste") and the "deepness" is allowed to stand out. The display screen may also display together information that a cooking example of the combination of the pork bone Chinese noodle soup and the bean paste is a pork bone bean paste Chinese noodle soup.

(Example 6)

The display screen 510 of the display apparatus 17 of the user side terminal 120 can similarly present (display) information on a combination having favorable compatibility of food-constituting materials (referred to as "food components").

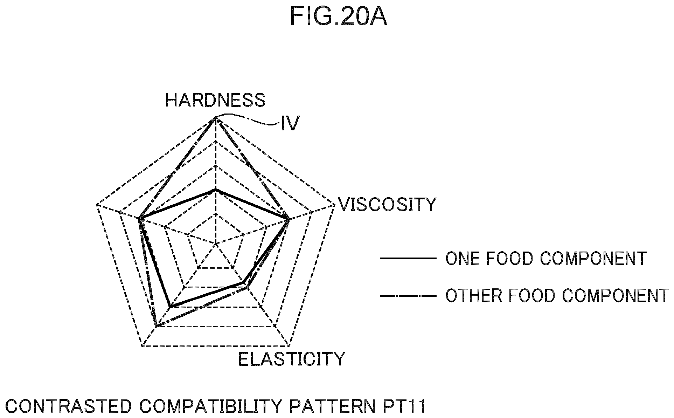

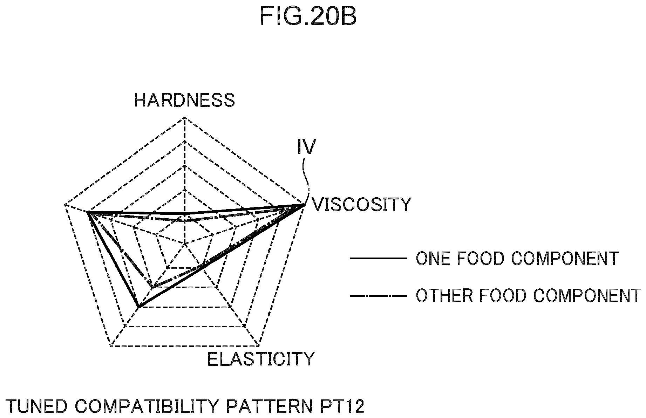

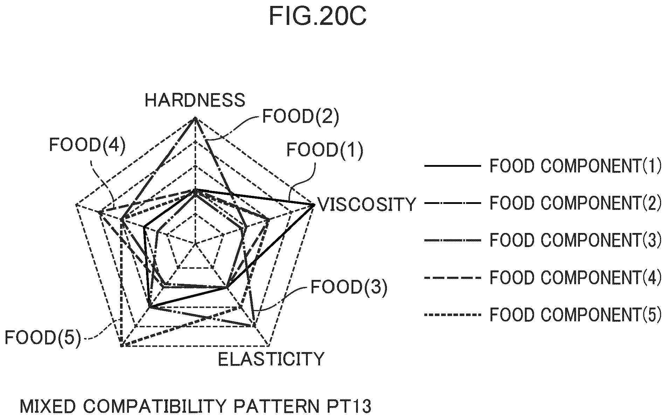

Figure 20A, Figure 20B, and Figure 20C each illustrate a pattern of a combination having favorable compatibility of food components.

Figure 20A illustrates a contrasted compatibility pattern PT11 in which physical properties of such food components are contrastive. Figure 20B illustrates a tuned compatibility pattern PT12 in which physical properties of such food components are resemblant to each other. Figure 20C illustrates a mixed compatibility pattern PT13 in which such food components have various physical properties. The mixed compatibility pattern PT13 has both the factor of the contrasted compatibility pattern PT11 and the factor of the tuned compatibility pattern PT12.

(Evaluation Item for Determination of Compatibility of Food Components)

A physical property value representing eating quality, in particular, a physical property value representing texture can be used for the evaluation item for determination of compatibility of food components.

Examples of the physical property value representing texture include hardness (soft/hard), viscosity (smooth/viscid), elasticity (resilience: low/high), roughness (slick/coarse), and crispy feeling (low/high).

In the present embodiment, a combination having favorable compatibility of food components is determined with multiple, three or more evaluation items including at least hardness, viscosity, and elasticity. A physical property value representing eating quality, for example, sound, the water content, or friction properties, can be used in the axis of an evaluation item other than "hardness", "viscosity", and "elasticity" in each radar chart illustrated in Figure 20A, Figure 20B, and Figure 20C.

A compatibility pattern storage section not illustrated stores each data of the contrasted compatibility pattern PT11, the tuned compatibility pattern PT12, and the mixed compatibility pattern PT13.

The contrasted compatibility pattern PT11, the tuned compatibility pattern PT12, and the mixed compatibility pattern PT13 each indicate a specific correspondence relationship between the evaluation values IV of food components considered to be favorably compatible with each other. The radar charts illustrated in Figure 20A, Figure 20B, and Figure 20C each illustrate each line joining the evaluation values IV of respective evaluation items "hardness", "viscosity", and "elasticity" of one food component, as a solid line, and each line joining the evaluation values IV of respective evaluation items "hardness", "viscosity", and "elasticity" of other food component, as a dash-dot-dash line.

· Contrasted Compatibility Pattern PT11

A correspondence relationship is meant in which matching or almost matching is obtained in terms of the evaluation values IV of two evaluation items (for example, "viscosity" and "elasticity") in the evaluation values IV of three evaluation items "hardness", "viscosity", and "elasticity", and a large deviation is observed with respect to the evaluation value IV of the remaining one evaluation item (for example, "hardness"). Other food component has the texture not provided by one food component and contrastive textures are obtained, whereby these components can be determined to provide a combination of food components favorably compatible with each other.

· Tuned Compatibility Pattern PT12

A correspondence relationship is meant in which matching or almost matching is obtained in terms of all the evaluation values IV of three evaluation items "hardness", "viscosity", and "elasticity". One food component and other food component

are resemblant in texture to each other and textures tuned are obtained, whereby these components can be determined to provide a combination of food components favorably compatible with each other.

· Mixed Compatibility Pattern PT13

A correspondence relationship is meant in which matching or almost matching is obtained in terms of all the evaluation values IV of three evaluation items "hardness", "viscosity", and "elasticity" and also a large deviation is observed with respect to all the evaluation values IV of three evaluation items "hardness", "viscosity", and "elasticity". Food components (1), (2), (3), (4) and (5) are combined and thus various textures are obtained, whereby these components can be determined to provide a combination of food components favorably compatible with each other.

(Exemplification of Contrasted Compatibility Pattern PT11)

Hereinafter, food components in a combination having favorable compatibility, corresponding to the contrasted compatibility pattern PT 11, are exemplified.
· A soft material and a hard material provide a combination having favorable compatibility. For example, a fried egg and a crispy bacon provide a combination having favorable compatibility.
A material favorable in meltability and a material remaining in the mouth provide a combination having favorable compatibility. For example, a confectionery combination of a potato chip and chocolate applied thereto is combination having favorable compatibility.

(Exemplification of Tuned Compatibility Pattern PT12)

Hereinafter, food components in a combination having favorable compatibility, corresponding to the tuned compatibility pattern PT12, are exemplified.
.Resilient materials are favorably compatible with each other. For example, a combination of pluck and konjac is a combination having favorable compatibility.
· Hard materials are favorably compatible with each other. For example, a combination of a cookie dough and a nut is a combination having favorable compatibility.
· Strongly sticking materials are favorably compatible with each other. For example, a combination of fermented soybean and okra is a combination having favorable compatibility.

(Exemplification of Mixed Compatibility Pattern PT13)

Hereinafter, food components in a combination having favorable compatibility, corresponding to the mixed compatibility pattern PT13, are exemplified.
A hamburger is exemplified. A hamburger is configured from buns having fluffy texture, a lettuce having crunchy texture, a tomato having juicy texture, a cheese having sticky texture, and a patty having resilient and loosed texture. A hamburger corresponds to a combination of materials having various textures, and provides a combination having favorable compatibility of food components, corresponding to the mixed compatibility pattern PT13.
Much the same is true on an apple pie. An apple pie is configured from a pie dough having crisp texture, apple compote having crunchy texture, and a custard having slick texture. An apple pie corresponds to a combination of materials having various textures, and provides a combination having favorable compatibility of food components, corresponding to the mixed compatibility pattern PT13.

(Presentation Example of Food Components Favorably Compatible with Each Other)

The display screen 510 of the display apparatus 17 of the user side terminal 120 can also similarly present (display) information on a combination having favorable compatibility of food components.

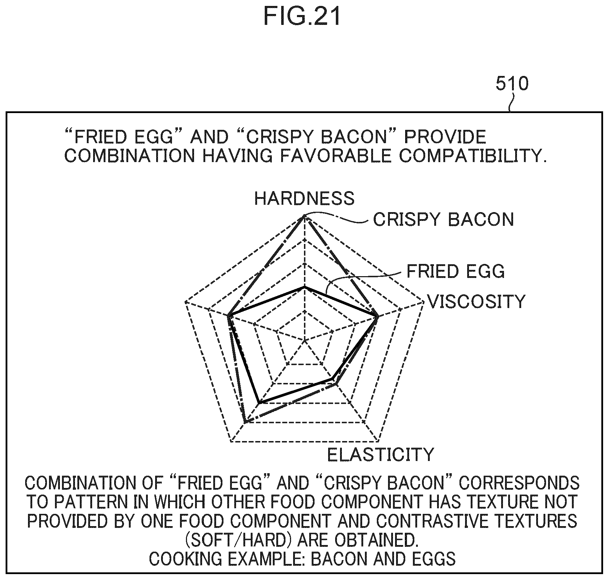

Figure 21 illustrates an example in which, in a case in which the presentation request of information on other food component going with a food component is made, the display screen 510 of the display apparatus 17 of the user side terminal 120 presents information on the item "fried egg" and the item "crispy bacon" determined to be favorably compatible with each other.

The item evaluation value IV of a food component is extracted from an evaluation value storage section not illustrated.

A correspondence relationship between the item evaluation value IV of the food component item "fried egg" and the item evaluation value IV of the food component item "crispy bacon" exhibits matching or almost matching in terms of the evaluation values IV of two evaluation items ("viscosity" and "elasticity") in the evaluation values IV of three evaluation items "hardness", "viscosity", and "elasticity", and a large deviation in terms of the evaluation value IV of the remaining one evaluation item ("hardness"). Thus, the correspondence relationship between the item evaluation value IV of the item "fried egg" and the item evaluation value IV of the item "crispy bacon" is determined to be the specific correspondence relationship indicated by the contrasted compatibility pattern PT11. Thus, the item "fried egg" and the item "crispy bacon" can be determined to provide a combination of food components favorably compatible with each other in that other food component has the texture not provided by one food component and contrastive textures (soft/hard) are obtained.

The display screen 510 of the display apparatus 17 of the user side terminal 120 not only displays information that the "fried egg" and the "crispy bacon" provide a combination having favorable compatibility, depending on the determination results, but also superposes and displays the item evaluation value IV of the food component "fried egg" and the item evaluation value IV of the food component "crispy bacon" on the radar chart of each evaluation item of "hardness", "viscosity", and "elasticity", as illustrated in Figure 21. The display screen may also display together information that the combination of the "fried egg" and the "crispy bacon" corresponds to a pattern in which other food component has the texture not provided by one food component and contrastive textures (soft/hard) are obtained. The display screen may also display together information on a cooking example (for example, "bacon and eggs") in which the item "fried egg" and the item "crispy bacon" are combined.

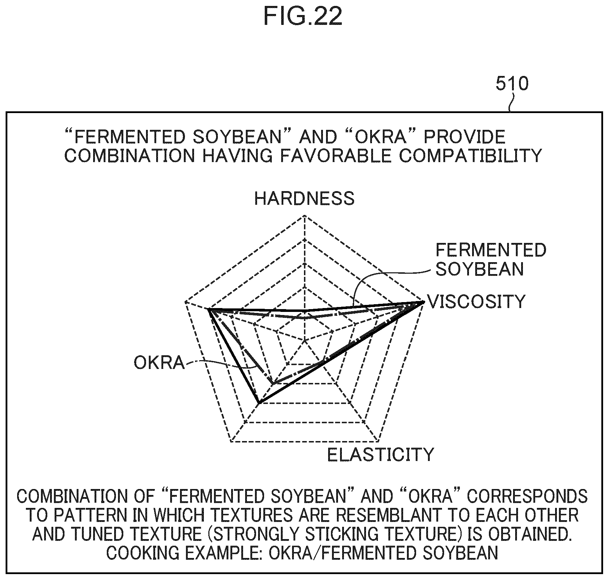

Figure 22 similarly illustrates an example in which, in a case in which the presentation request of information on other food component going with a food component is made, the display screen 510 of the display apparatus 17 of the user side terminal 120 presents information on the item "fermented soybean" and the item "okra" determined to be favorably compatible with each other.

The item evaluation value IV of a food component is extracted from an evaluation value storage section not illustrated.

A correspondence relationship between the item evaluation value IV of the food component item "fermented soybean" and the item evaluation value IV of the food component item "okra" is a correspondence relationship in which matching or almost matching is obtained in terms of all the evaluation values IV of three evaluation items "hardness", "viscosity", and "elasticity". A correspondence relationship is obtained in which the "viscosity" is high and sticking is tuned therewith at a high level. Thus, the item "fermented soybean" and the item "okra" can be determined to provide a combination having favorable compatibility of food components in that respective textures of such food components are resemblant to each other and the tuned texture (strongly sticking texture) is obtained.

The display screen 510 of the display apparatus 17 of the user side terminal 120 not only displays information that the "fermented soybean" and the "okra" provide a combination having favorable compatibility, depending on the determination result, but also superposes and displays the item evaluation value IV of the food component "fermented soybean" and the item evaluation value IV of the food component "okra" on the radar chart of each evaluation item of "hardness", "viscosity", and "elasticity", as illustrated in Figure 22. The display screen may also display together information that a combination of the "fermented soybean" and the "okra" corresponds to a pattern in which textures are resemblant to each other and the tuned texture (strongly sticking texture) is obtained. The display screen may also display together information on a cooking example (for example, "okra/fermented soybean") of a combination of the item "fermented soybean" and the item "okra".

Similarly, in a case in which the presentation request of the information on other food component going with a food component is made, the display screen 510 of the display apparatus 17 of the user side terminal 120 can display information that the food components "bun", "lettuce", "tomato", "cheese", and "patty" are food components favorably compatible with one another, and also can together display information that a combination of such food components corresponds to a pattern (mixed compatibility pattern PT13) imparting various textures and information that a cooking example is, for example, "hamburger".

(Compatibility Determination Method by Principal Component Analysis)

While the case of determination of compatibility of food components by use of compatibility patterns is described as an example, compatibility of food components may be determined by principal component analysis of many physical property values of food components.

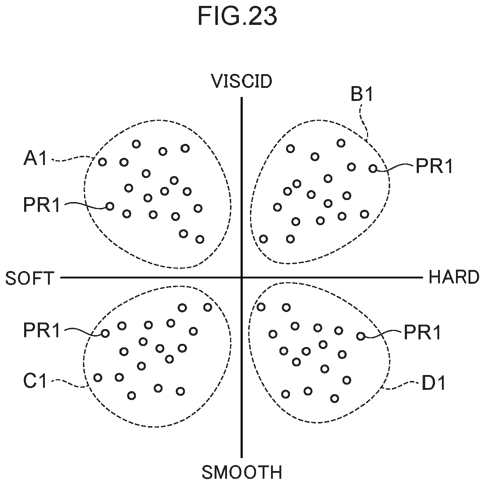

Figure 23 is a graph with two evaluation axes of a horizontal axis representing the physical property value of "soft/hard" and a vertical axis representing the physical property value of "smooth/viscid". Figure 23 illustrates a scattering diagram in which respective evaluation values of "soft/hard" and "smooth/viscid" with respect to each item of food components are plotted at coordinate positions on the evaluation axes.

Many physical property values of hardness, viscosity, elasticity, and the like of food components are subjected to principal component analysis, and these many physical property values are collectively synthesized on two respective evaluation axes of "soft/hard" and "smooth/viscid". The respective evaluation values of "soft/hard" and "smooth/viscid" with respect to each of food component items are acquired, and are plotted on the scattering diagram of Figure 23. Each coordinate point plotted is defined as PR1.

As food component items are closer to each other in terms of coordinate point PR1 plotted on the scattering diagram of Figure 23, these food components are determined to be favorably compatible with each other. In one example, food components belonging to "viscid and soft" A1 group are determined to be favorably compatible with each other. Food components belonging to "viscid and hard" B 1 group are determined to be favorably compatible with each other. Food components belonging to "smooth and soft" C1 group are determined to be favorably compatible with each other. Food components belonging to "smooth and hard" D1 group are determined to be favorably compatible with each other.

(Example 7)

(Compatibility Determination by Number of Common Volatile Flavor Components)

Next, each method of determining compatibility of a drink and a food, compatibility of cuisine and a seasoning, compatibility of seasonings, and compatibility of food components by use of an odor component is described.

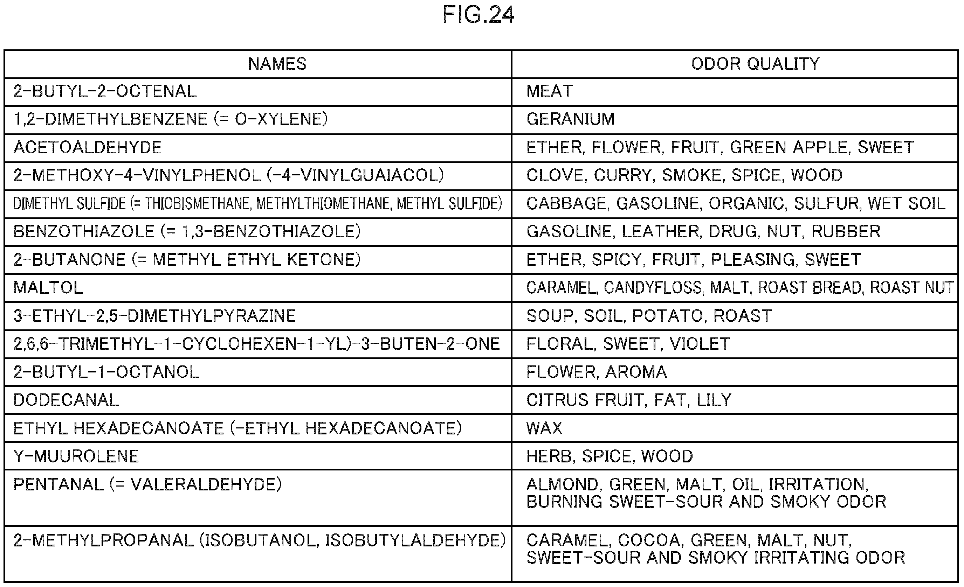

Examples of the odor component include various kinds of volatile flavor components. Figure 24 illustrates correspondence relationships between various kinds of volatile flavor components and odor qualities. For example, the volatile flavor component "acetoaldehyde" contributes to the odor quality "ether, flower, fruit, green apple, sweetness".

As the number of common volatile flavor components between compatibility determination subjects (for example, a drink and a food) is larger, namely, the number of odor components is larger, such subjects can be determined to be favorably compatible with each other in that these are common in terms of the odor quality.

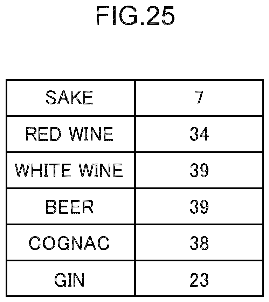

A case of determination of compatibility of a citrus iyo peel and an alcoholic drink is exemplified. Figure 25 represents the number of common volatile compounds between each of sake, red wine, white wine, beer, cognac, and gin, and a citrus iyo peel.

White wine and beer each have a large number of common volatile compounds, of 39, as compared with other categories of alcoholic drinks (sake, red wine, cognac, gin), and can be determined to be each favorably compatible with citrus iyo peel, as compared with such other categories of alcoholic drinks.

(Compatibility Determination Method by Principal Component Analysis)

Compatibility of a drink and a food, compatibility of cuisine and a seasoning, compatibility of seasonings, and compatibility of food components may also be determined by principal component analysis of output peak values assigned to various kinds of components, output from an odor component analyzer.

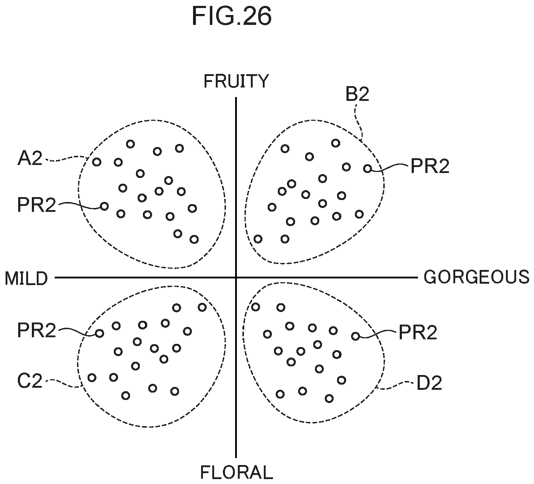

Figure 26 is a graph with two evaluation axes in which a horizontal axis represents the evaluation value "mild/gorgeous" and a vertical axis represents the evaluation value "floral/fruity". Figure 26 illustrates a scattering diagram in which respective evaluation values of "mild/gorgeous" and "floral/fruity" with respect to each of, for example, drink and food items are plotted at coordinate positions on the evaluation axes.

Peak values assigned to various kinds of volatile flavor components, output from an odor component analyzer, are subjected to principal component analysis, and such many values of volatile flavor components are collectively synthesized on two respective evaluation axes of "mild/gorgeous" and "floral/fruity". The respective evaluation values of "mild/gorgeous" and "floral/fruity" with respect to each of drink and food items are acquired, and are plotted on the scattering diagram of Figure 26. Each coordinate point plotted is defined as PR2.

As drink and food items are closer to each other in terms of coordinate point PR2 plotted on the scattering diagram of Figure 26, such drink and food are determined to be favorably compatible with each other. In one example, a drink and a food belonging to "fruity and mild" A2 group are determined to be favorably compatible with each other. A drink and a food belonging to "fruity and gorgeous" B2 group are determined to be favorably compatible with each other. A drink and a food belonging to "floral and mild" C2 group are determined to be favorably compatible with each other. A drink and a food belonging to "floral and gorgeous" D2 group are determined to be favorably compatible with each other. The same can also apply to determination of compatibility of cuisine and a seasoning, determination of compatibility of seasonings, and determination of compatibility of food components.

(Compatibility Determination Method by Setting Of Reference Smell Range)

The range from the acceptable maximum value to the acceptable minimum value of the evaluation value IV of odor is set as a reference smell range SZ in advance, and, in a case in which the evaluation values IV of compatibility determination subjects (for example, a drink and a food) each fall within the reference smell range SZ, these subjects may be determined to be favorably compatible with each other.

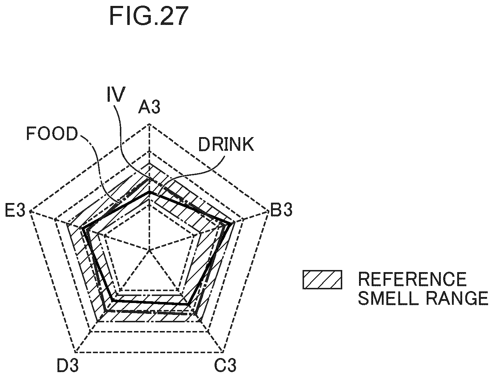

Figure 27 illustrates the evaluation value IV of each evaluation item (evaluation item A3, evaluation item B3, evaluation item C3, evaluation item D3, and evaluation item E3) of odor on a radar chart. The evaluation items A3, B3, C3, D3, and E3 can respectively correspond to sulfur-based, hydrocarbon-based, aromatic, ester-based, and aldehyde-based odor components, as in the illustration of Figure 4B. The evaluation items A3, B3, C3, D3, and E3 may also correspond to volatile flavor components.

The radar chart illustrated in Figure 27 illustrates each line joining the evaluation values IV of the evaluation items A3, B3, C3, D3, and E3 of a drink, as a solid line, and each line joining the evaluation values IV of the evaluation items A3, B3, C3, D3, and E3 of a food, as a dash-dot-dash line.

The reference smell range SZ is set with respect to each of the evaluation items A3, B3, C3, D3, and E3. The reference smell range SZ is a range from the acceptable minimum value to the acceptable maximum value with respect to odor which feels pleasant to people.

In a case in which each of the evaluation values IV of the evaluation items A3, B3, C3, D3, and E3 of a drink falls within the reference smell range SZ and each of the evaluation values IV of the evaluation items A3, B3, C3, D3, and E3 of a food falls within the reference smell range SZ, each odor of such drink and food falls within a range which feels pleasant to people and such drink and food are determined to provide a combination having favorable compatibility. The same can also apply to determination of compatibility of cuisine and a seasoning, determination of compatibility of seasonings, and determination of compatibility of food components.

(Example 8)

A method of diagnosing compatibility of a drink and a food by diagnosing compatibility of a drink and a food by use of a detection value in a taste sensor is described.

The method of the present Example involves diagnosing compatibility of a drink and a food based on the aftertaste of a food due to flushing with a drink.

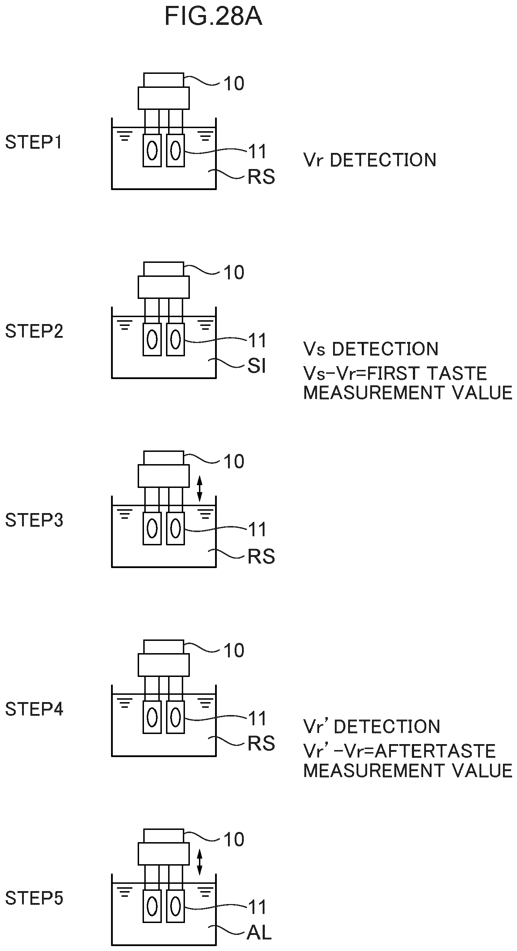

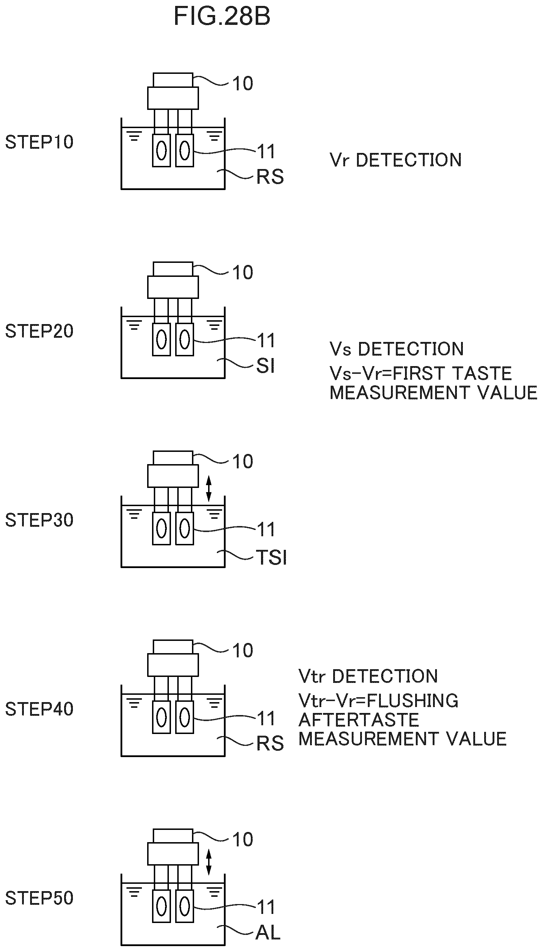

Figure 28A illustrates the procedure of Comparative Example as compared with the present Example, and illustrates a procedure for measurement of the aftertaste of a food with a taste sensor 10. Figure 28B illustrates the procedure of the present Example, and illustrates a procedure for measurement of the aftertaste of a food due to flushing with a drink with a taste sensor 10 as compared with Figure 28A.

The taste sensor 10 for use in the Example is a sensor capable of measuring the "first taste" as a taste at the moment of putting of a food in the mouth and the "aftertaste" as a taste having sustention remaining after swallowing of such a food. The "aftertaste" is expressed by, for example, astringency, bitterness, or richness. A reference liquid RS is used in order to immerse a sensor section 11 of the taste sensor 10 in a liquid and detect a reference potential Vr corresponding to "tasteless". The reference liquid RS is a liquid having a composition corresponding to that of human saliva, and, for example, a mixed solution of potassium chloride KCl and tartaric acid at a predetermined ratio is used.

(Comparative Example: Measurement of Aftertaste of Food)

Step 1: immersing the sensor section 11 of the taste sensor 10 in the reference liquid RS and detecting the reference potential Vr, as illustrated in Figure 28A.

Step 2: next, immersing the sensor section 11 in a food sample liquid SI and detecting a potential Vs. The measurement value of the first taste is obtained from the following expression (1).

$$Vs - Vr = \text{Measurement value of first taste} \dots (1)$$

Step 3: next, simply washing the sensor section 11 with the reference liquid RS. The simply washing is not to fully wash the sensor section 11, but to wash the sensor section 11 to such an extent that a component of the food sample liquid SI slightly remains on the sensor section. The sensor section 11 is moved up and down, and the food sample liquid SI attached to the sensor section 11 is flushed with the reference liquid RS.

Step 4: immersing the sensor section 11 in the reference liquid RS and detecting a reference potential Vr', as in step 1. The measurement value of the aftertaste is obtained from the following expression (2).

$$Vr' - Vr = \text{Measurement value of aftertaste} \dots (2)$$

Step 5: immersing the sensor section 11 in an alcohol solution AL, and fully washing the sensor section 11 for refreshing. Preparation of the next measurement is completed, and transfer to step 1 can be made.

(Example: Measurement of Aftertaste of Food Due to Flushing with Drink, and Compatibility Diagnosis)

In the Example, the sensor section 11 is washed with a drink TSI as a compatibility diagnosis target, instead of washing of the sensor section 11 with the reference liquid RS. Hereinafter, a method of diagnosing compatibility, including step 10 to step 60, is described.

Step 10: immersing the sensor section 11 of the taste sensor 10 in the reference liquid RS and detecting the reference potential Vr, as illustrated in Figure 28B.

Step 20: next, immersing the sensor section 11 in a food sample liquid SI and detecting a potential Vs. The measurement value of the first taste is obtained from the following expression (1).

$$Vs - Vr = \text{Measurement value of first taste} \dots (1)$$

Step 30: next, simply washing the sensor section 11 with the drink TSI as a compatibility diagnosis target. The sensor section 11 is moved up and down, and the food sample liquid SI attached to the sensor section 11 is flushed with the drink TSI as a compatibility diagnosis target.

Step 40: immersing the sensor section 11 in the reference liquid RS and detecting a reference potential Vtr', as in step 10. The measurement value of the aftertaste of a food due to flushing with a drink is obtained from the following expression (3).

$$Vtr' - Vr = \text{Measurement value of aftertaste of a food due to flushing with a drink} \dots (3)$$

Step 50: immersing the sensor section 11 in an alcohol solution AL, and fully washing the sensor section 11 for refreshing. Preparation of the next measurement is completed, and transfer to step 10 can be made.

Step 60: diagnosing compatibility of a drink and a food depending on the measurement value Vtr' - Vr (evaluation value IV of umami-richness) of the aftertaste of a food due to flushing with a drink, measured as described above.

(Compatibility Diagnosis Example with Evaluation Value of Aftertaste of Food Due to Flushing with Drink)

An example of diagnosis of compatibility of sushi (tuna) and sake is described.

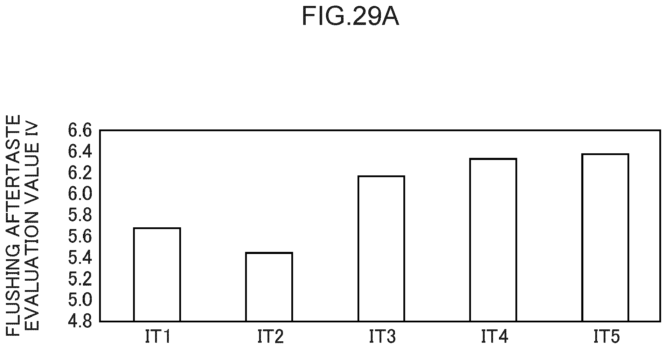

Figure 29A is a graph illustrating the numerical value of an evaluation value (hereinafter, referred to as "flushing aftertaste evaluation value") IV of aftertaste of sushi (tuna) due to flushing with each of five brands IT1, IT2, IT3, IT4, and IT5 of sakes. The flushing aftertaste evaluation value IV can be expressed by umami or richness. It is estimated that, as the flushing aftertaste evaluation value IV is lower, the degree of flushing of sushi (tuna) with sake is higher and sake is more enjoyed. It is estimated that, as the flushing aftertaste evaluation value IV is higher, the degree of flushing of sushi (tuna) with sake is lower, and the aftertaste of sushi (tuna) more remains in the mouth, thereby resulting in more tuning between sake and sushi (tuna) and a higher sense of satisfaction in meal.

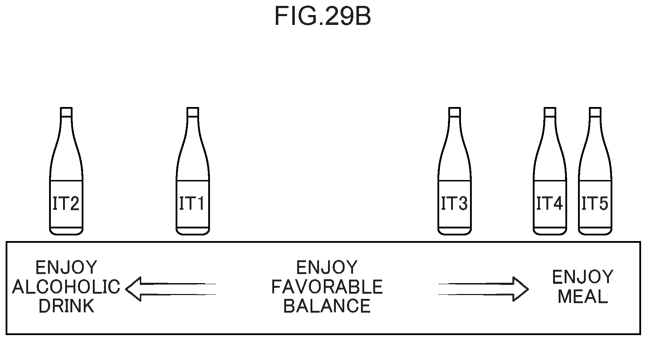

Figure 29B illustrates the compatibility diagnosis results by use of the flushing aftertaste evaluation value IV

The flushing aftertaste evaluation value IV of the brand IT2 of sake is the lowest value, and the compatibility diagnosis result "enjoying of an alcoholic drink (high degree of flushing)" is obtained in a combination of the brand IT2 of sake and the sushi (tuna).

The flushing aftertaste evaluation value IV of the brand IT1 of sake is the second lowest value, and the compatibility diagnosis result "enjoying of an alcoholic drink and meal in well-balanced manner (moderate degree of flushing)" closer to "enjoying of an alcoholic drink (high degree of flushing)" is obtained in a combination of the brand IT1 of sake and the tuna.

The flushing aftertaste evaluation value IV of the brand IT3 of sake is the third highest value, and the compatibility diagnosis result "enjoying of an alcoholic drink and meal in well-balanced manner (moderate degree of flushing)" closer to "enjoying of meal (low degree of flushing)" is obtained in a combination of the brand IT3 of sake and the tuna.

The flushing aftertaste evaluation value IV of the brand IT4 of sake is the second highest value, and the compatibility diagnosis result "enjoying of meal (low degree of flushing)" is obtained in a combination of the brand IT4 of sake and the tuna.

The flushing aftertaste evaluation value IV of the brand IT5 of sake is the highest value, and the compatibility diagnosis result "enjoying of meal (low degree of flushing)" is obtained in a combination of the brand IT5 of sake and the tuna.

(Compatibility Diagnosis Example with Flushing Aftertaste Evaluation Value of Drink Other Than Sake)

A drink other than sake, for example, tea (green tea) or soft drink (coke) can also be subjected to compatibility diagnosis with the flushing aftertaste evaluation value IV

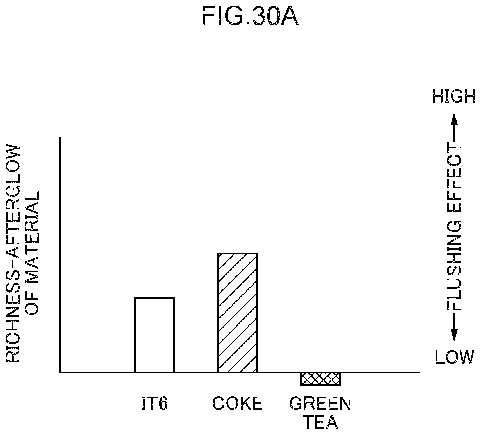

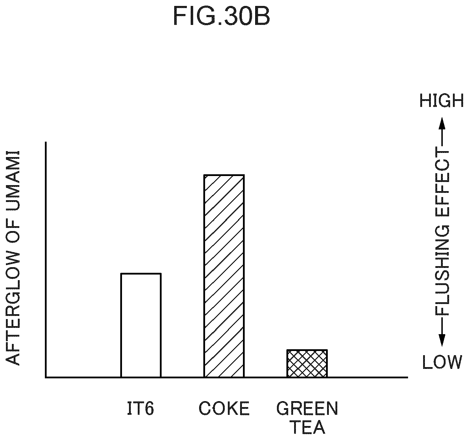

Figures 30A and 30B are each a graph illustrating the flushing aftertaste evaluation value IV in the case of a combination of pizza with each of sake, tea (green tea), and soft drink (coke).

The aftertaste of a food due to flushing with a drink can be expressed by "richness, afterglow of material feeling" or "afterglow of umami". Figure 30A illustrates the flushing aftertaste evaluation value IV of "richness, afterglow of material feeling". Figure 30B illustrates the flushing aftertaste evaluation value IV of "afterglow of umami".

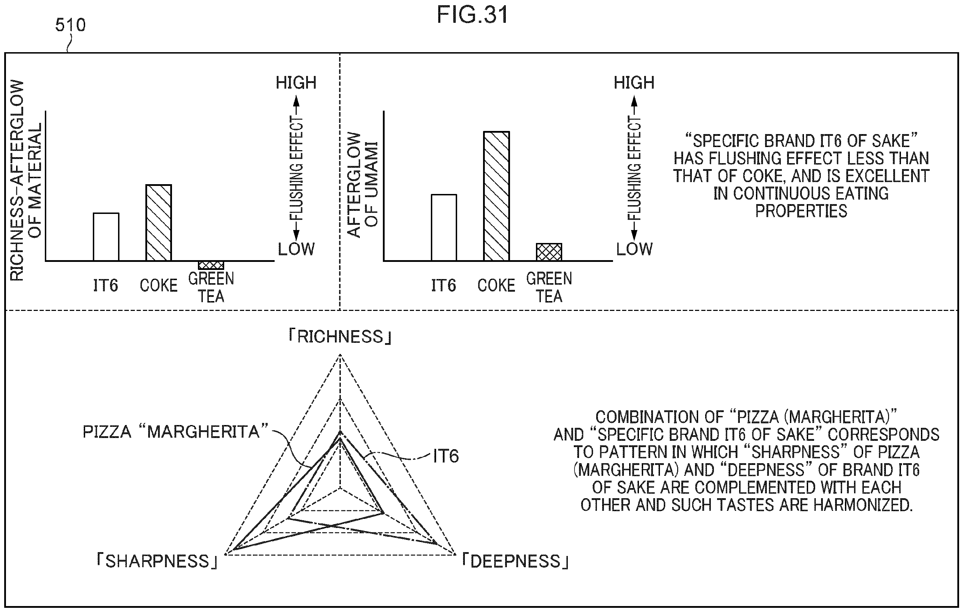

Figure 31 illustrates an example in which, in a case in which the presentation request of information on a drink going with a food is made, the display screen 510 of the display apparatus 17 of the user side terminal 120 presents information on the food item "pizza (Margherita)" and the drink item "specific brand IT6 of sake" determined to be favorably compatible with each other. It can be seen that the "specific brand IT6 of sake" has a flushing effect less than that of coke, and is excellent in continuous eating properties.

The display screen 510 of the display apparatus 17 of the user side terminal 120 displays the flushing aftertaste evaluation value IV of the food "pizza (Margherita)" by the drink "specific brand IT6 of sake". The display screen may display the flushing aftertaste evaluation value IV of the specific brand IT6 of sake comparatively with the flushing aftertaste evaluation values IV of tea (green tea) and soft drink (coke). The display screen may also display information that the "specific brand IT6 of sake" has a flushing effect less than that of coke, and is excellent in continuous eating properties.

The display screen may also display together a radar chart R16 in which the respective item evaluation values IV of richness, sharpness, and deepness of each of the food "pizza (Margherita)" and the drink "specific brand IT6 of sake" are superposed. The display screen may also display together information that the combination of the "pizza (Margherita)" and the "specific brand IT6 of sake" corresponds to a pattern (fourth compatibility pattern PT4) in which "sharpness" of the pizza (Margherita) and "deepness" of the specific brand IT6 of sake are complemented with each other and such tastes are harmonized.

(Example 9)

The number of evaluation items for use in compatibility determination is any number, and may be, for example, three (three axes) or may be, for example, six (six axes).

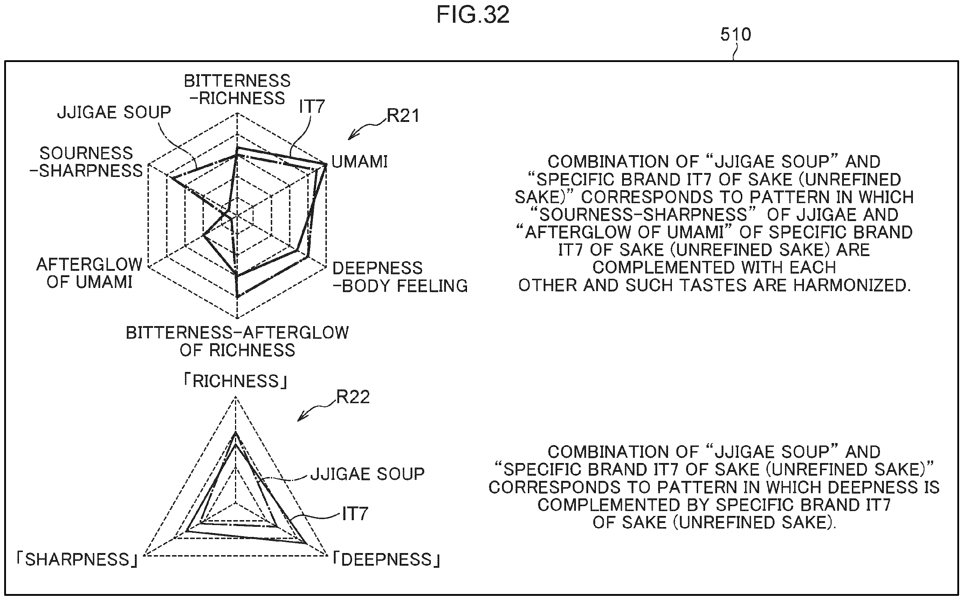

Figure 32 illustrates an example displaying both a radar chart R21 in which item evaluation values IV of a specific brand IT7 of sake (unrefined sake) and six evaluation items of jjigae soups (six axes) are superposed, and a radar chart R22 in which item evaluation values IV of a specific brand IT7 of sake (unrefined sake) and three evaluation items of jjigae soups (three axes) are superposed.

The radar chart R21 is configured by six (six axes) evaluation items of bitterness-richness, sourness-sharpness, afterglow of umami, bitterness-afterglow of richness, deepness-body feeling, and umami. The radar chart R22 is configured by three (three axes) evaluation items of richness, sharpness, and deepness.

In a case in which the presentation request of the information on a food going with a drink or the presentation request of the information on a drink going with a food is made, the display screen 510 of the display apparatus 17 of the user side terminal 120 displays information on the food item "jjigae soup" and the drink item "specific brand IT7 of sake (unrefined sake)" determined to be favorably compatible with each other.

The display screen 510 of the display apparatus 17 of the user side terminal 120 displays a hexaxial radar chart R21. The display screen may also display together information that the combination of the "jjigae soup" and the "specific brand IT7 of sake (unrefined sake)" corresponds to a pattern in which "sourness-sharpness" of the jjigae soup and "afterglow of umami" of the specific brand IT7 of sake (unrefined sake) are complemented with each other and such tastes are harmonized.

The display screen 510 of the display apparatus 17 of the user side terminal 120 displays a triaxial radar chart R22, together with the hexaxial radar chart R21. The display screen may also display together information that the combination of the "jjigae soup" and the "specific brand IT7 of sake (unrefined sake)" corresponds to a pattern (third compatibility pattern PT3) in which deepness is complemented by the specific brand IT7 of sake (unrefined sake).

(Example 10)

It is also possible to perform presentation of a drink and a food favorably compatible with each other, cuisine and a seasoning favorably compatible with each other, seasonings favorably compatible with each other, and food components favorably compatible with each other to a user with information on drinking and eating history of the user. It is also possible to further perform presentation of a drink and a food favorably compatible with each other, cuisine and a seasoning favorably compatible with each other, seasonings favorably compatible with each other, and food components favorably compatible with each other to the user within the calorie range of meal which can be taken today.

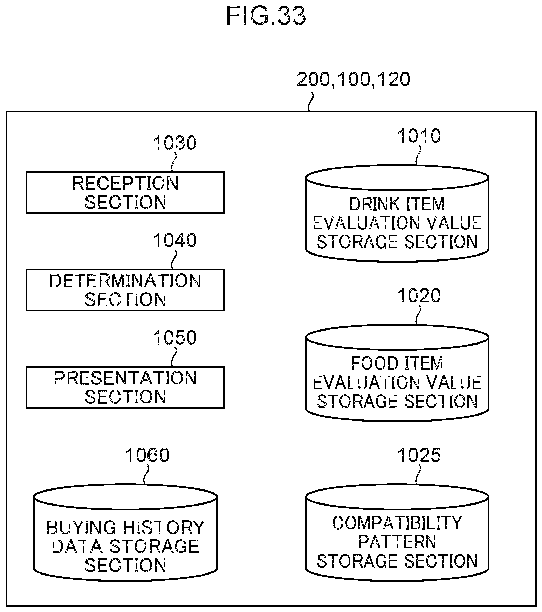

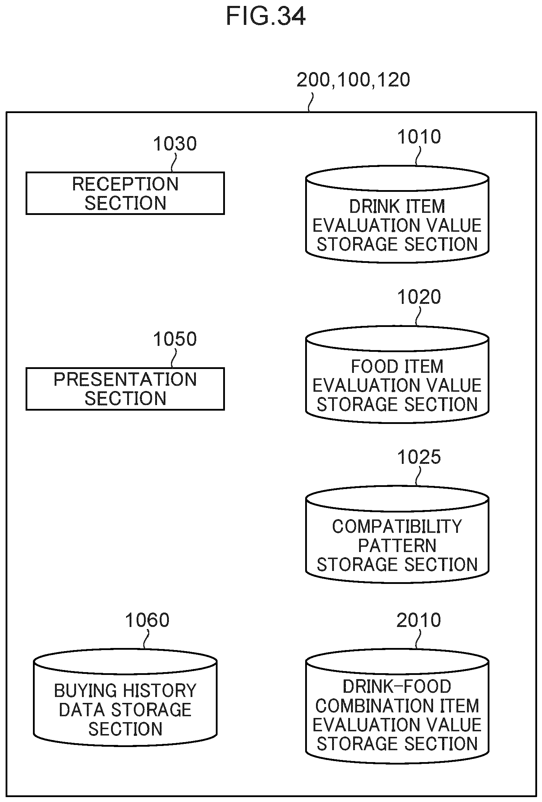

Figure 33 and Figure 34 are block diagrams illustrating functional configurations of presentation apparatuses of information on compatibility of Examples corresponding to Figure 3 and Figure 14. Each of the functional configurations illustrated in Figure 33 and Figure 34 includes a server 200, an administrator side terminal 100, and a user side terminal 120. A buying history data storage section 1060 is added as compared with Figure 3 or Figure 14. The buying history data storage section 1060 stores user-by-user history data representing drink, food, seasoning, and/or food component item(s) ever bought by or provided to a user, in association with the user.

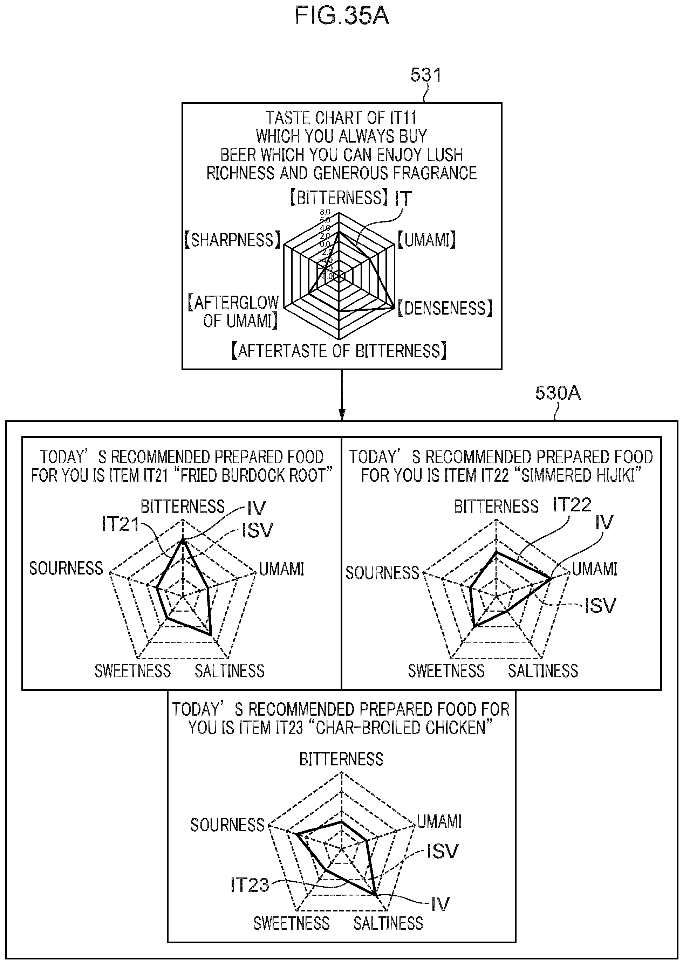

Figure 35A illustrates a compatibility information presentation screen 530A displayed on a display screen of a user side terminal 120 of a user ID1, according to a request of presentation of "prepared food item favorably compatible with a drink ever bought by the user ID1".

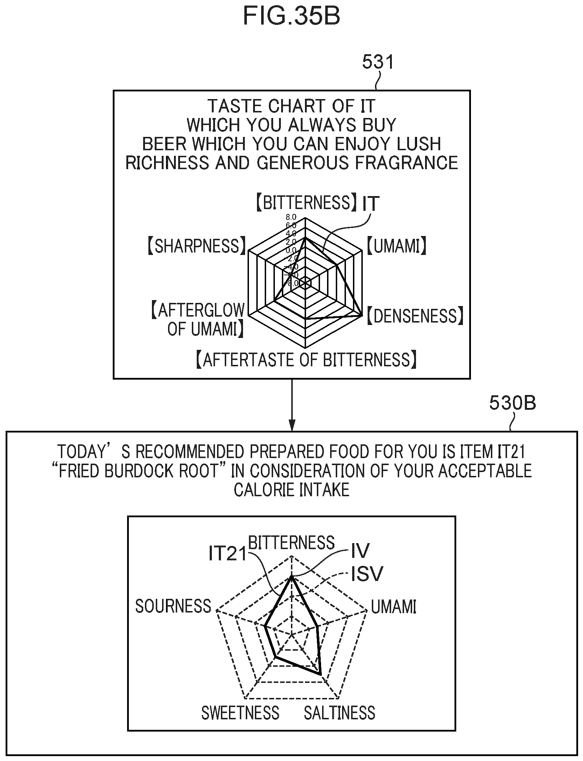

For example, a tablet terminal mounted to a shopping cart of a store STO1 (supermarket) can be adopted as the user side terminal 120. The store STO1 has control competence, for example, for the change in content to be displayed on compatibility information presentation screens 530A and 530B, and a buying history screen 531 illustrated in Figures 35A and 35B, through a control screen not illustrated of the store side terminal 120. For example, the store STO1 can limit an item to be presented on the compatibility information presentation screens 530A and 530B, to a commercial product item which can be handled in the store STO1.

A reception section 1030 can acquire a user ID of a user who performs a login operation, namely, a user ID1, according to the login operation in the user side terminal 120.

A buying history is here associated with each user ID. User-by-user buying history data UD includes the listing of the user ID, the listing of the item IT(trade name) bought in a shop or the like or subjected to service provision in an eating establishment, and the listing of a purchase quantity (purchase price). A buying history data storage section 1060 of a server 200 stores the user-by-user buying history data UD. The buying history data storage section 1060 stores calorie data CD. The calorie data CD is data of an acceptable calorie intake for a user and the calorie of a commercial product purchased or subjected to service provision.

For example, in a case in which the user ID1 performs a login operation in the user side terminal 120, the data of an item IT 11 in the category of the beer ever bought by the user ID1 is extracted with reference to the user-by-user buying history data UD stored in the buying history data storage section 1060. The data of the item evaluation value IV in association with the item IT11 is extracted from the drink item evaluation value storage section 1010. The buying history screen 531 is displayed on the display screen of the user side terminal 120, based on the data of the item evaluation value IV associated with the item IT 11 extracted.

The buying history screen 531 displays information on "taste chart of IT11 which you always buy, beer which you can enjoy lush richness and generous fragrance" and also displays information on the radar chart of the item evaluation value IV associated with the item IT11.

A case in which a second embodiment in Figure 34 is applied is exemplified.

The data of "prepared food" favorably compatible with the item IT11 "specific beer" is extracted from a drink-food combination item evaluation value storage section 2010. The item IT21 "fried burdock root", the item IT22 "simmered hijiki", and the item IT23 "char-broiled chicken", as the prepared foods favorably compatible with the item IT11 "specific beer", are extracted as presentation candidates to the user ID1. As a result, the display screen of the display apparatus 17 of the user side terminal 120 displays information on "today's recommended prepared food for you is item IT21 "fried burdock root"", as illustrated in Figure 35A, and also displays information on the radar chart of the item evaluation value IV associated with the item IT21. The display screen displays information on "item IT22 "simmered hijiki" is also recommend", and also displays information on the radar chart of the item evaluation value IV associated with the item IT22.

The display screen displays information on "item IT23 "char-broiled chicken" is also recommend", and also displays information on the radar chart of the item evaluation value IV associated with the item IT23.

Thus, buying of not only the "specific beer" ever bought by the user ID1, but also "fried burdock root", "simmered hijiki", and/or "char-broiled chicken" favorably compatible with the "specific beer" can be promoted.

A recommended prepared food providing a calorie intake in an acceptable range for a user may be presented among the "fried burdock root", the "simmered hijiki", and the "char-broiled chicken".

The acceptable calorie intake for a user is a value obtained by subtracting the calorie value taken actually heretofore for one day, from the required amount of energy (for example, 2700 kcal for adult men) per day depending on the attribute of a user. The buying history data storage section 1060 stores the required amount of energy per one day depending on the attribute of a user and the calorie value taken actually heretofore for one day.

The calorie value taken actually heretofore for one day can be acquired according to automatic calculation by registering the meal content in association with the user ID of a user. The calorie value can also be acquired by measuring the calorie intake taken, with a sensor section and a computing section provided in a wearable terminal, a smart watch, or a smart band worn by a user. The calorie value taken actually heretofore for one day, here acquired, is sent to the buying history data storage section 1060 in the server 200.

In this regard, the calorie value with respect to each item IT is memorized in the drink item evaluation value storage section 1010 and the food item evaluation value storage section 1020, in association with such each item IT.

For example, in a case in which the remaining acceptable calorie intake per day for a user is 500 kcal, only a combination of the "specific beer" (500 cc) and the "fried burdock root" (200 g) has less than 500 kcal and satisfies a condition of less than the acceptable calorie intake, and a combination of the specific beer" (500 cc) and the "simmered hijiki" (200 g) and a combination of the "specific beer" (500 cc) and the "char-broiled chicken" (200 g) each have more than 500 kcal and do not satisfy a condition of less than the acceptable calorie intake. In a case in which the weight (volume) per package differs depending on the food item or the weight differs with respect to each package even in the case of the same food item, the calorie value may be associated with respect to each package of a food.

As a result, the compatibility information presentation screen 530B of the display apparatus 17 of the user side terminal 120 displays information on "today's recommended prepared food for you is item IT21 "fried burdock root" in consideration of your acceptable calorie intake", and also displays information on the radar chart of the item evaluation value IV associated with the item IT21, as illustrated in Figure 35B.

Thus, buying of not only the "specific beer" ever bought by the user ID1, but also the "fried burdock root" favorably compatible with the "specific beer" can be promoted within an acceptable calorie intake range.

A case in which a food favorably compatible with the drink ever bought by a user in a store such as a supermarket is presented is exemplified and described above.

However, the same manner may be performed even in a case in which a food favorably compatible with the drink ever subjected to service provision to a user in a store such as a restaurant is presented.

The same manner may be performed even in a case in which a drink favorably compatible with the food ever bought by a user in a store is presented, or even in a case in which a drink favorably compatible with the food ever subjected to service provision to a user in a store is presented.

The same manner may be performed even in a case in which a seasoning favorably compatible with the cuisine ever bought by a user in a store is presented, or even in a case in which a seasoning favorably compatible with the cuisine ever subjected to service provision to a user in a store is presented.

The same manner may be performed even in a case in which other seasoning favorably compatible with the seasoning ever bought by a user in a store is presented, or even in a case in which a seasoning favorably compatible with the seasoning ever subjected to service provision to a user in a store is presented.

The same manner may be performed even in a case in which other food component favorably compatible with the food component ever bought by a user in a store is presented, or even in a case in which other food component favorably compatible with the food component ever subjected to service provision to a user in a store is presented.

The disclosure of Japanese Patent Application No. 2021-81158 filed on May 12, 2021 is herein incorporated by reference in its entirety.

All literatures, patent applications, and technical standards described herein are herein incorporated by reference, as if individual literature, patent application, and technical standard were specifically and individually indicated to be incorporated by reference.

Reference Signs List

1010 drink item evaluation value storage section
1020 food item evaluation value storage section

1025 compatibility pattern storage section
1030 reception section
1040 determination section
1050 presentation section
1060 buying history data storage section
2010 drink-food combination item evaluation value storage section

**Claims**

1. An apparatus for presenting information regarding compatibility between a drink and a food, the apparatus comprising:

   a drink item evaluation value storage section in which an item evaluation value for each of a plurality of evaluation items for determination of compatibility with a food in terms of one or a combination of two or more of taste, odor, and eating quality, in association with a drink item and a food category of a pairing target, is stored as a relative numerical value with respect to an item reference value;
   a food item evaluation value storage section in which an item evaluation value for each of the plurality of evaluation items for determination of compatibility with a drink in terms of one or a combination of two or more of taste, odor, and eating quality, in association with a food item and a drink category of a pairing target, is stored as a relative numerical value with respect to an item reference value;
   a reception section that receives a request for presentation of information regarding a combination having favorable compatibility between a drink and a food;
   a determination section that extracts item evaluation values of a drink and a food in the combination according to the presentation request received by the reception section, from each of the drink item evaluation value storage section and the food item evaluation value storage section, and, in a case in which a correlation between the extracted item evaluation values for the drink and the food is matched to a specific correlation regarded as resulting in favorable compatibility, determines the drink and the food to be a combination having favorable compatibility; and
   a presentation section that presents information regarding the drink and the food determined by the determination section to be the combination having favorable compatibility.

2. The apparatus for presenting information regarding compatibility between a drink and a food according to claim 1, wherein the presentation section superposes and displays the item evaluation value of the drink and the item evaluation value of the food on a radar chart of the plurality of evaluation items.

3. The apparatus for presenting information regarding compatibility between a drink and a food according to claim 1 or 2, wherein:

   the specific correlation regarded as resulting in favorable compatibility is prepared in the form of a plurality of compatibility patterns respectively exhibiting different correlations, and
   the presentation section presents information regarding a correlation between item evaluation values of a drink and a food determined to be a combination having favorable compatibility, with respect to each of the plurality of compatibility patterns.

4. An apparatus for presenting information regarding compatibility between a drink and a food, the apparatus comprising:

   a drink item evaluation value storage section in which an item evaluation value for each of a plurality of evaluation items for determination of compatibility with a food in terms of one or a combination of two or more of taste, odor, and eating quality, in association with a drink item and a food category of a pairing target, is stored as a relative numerical value with respect to an item reference value;
   a food item evaluation value storage section in which an item evaluation value for each of the plurality of evaluation items for determination of compatibility with a drink in terms of one or a combination of two or more of taste, odor, and eating quality, in association with a food item and a drink category of a pairing target, is stored as a relative numerical value with respect to an item reference value;
   a drink-food combination item evaluation value storage section in which item evaluation values of a drink and a food in a specific combination, in which a correlation between item evaluation values of a drink and a food stored in the drink item evaluation value storage section and the food item evaluation value storage section is matched to a specific correlation regarded as resulting in favorable compatibility, are stored;

a reception section that receives a request for presentation of information regarding a combination having favorable compatibility between a drink and a food; and

a presentation section that extracts item evaluation values for a drink and a food in the combination according to the presentation request received by the reception section, from the drink-food combination item evaluation value storage section, and presents information regarding the drink and the food extracted.

5. The apparatus for presenting information regarding compatibility between a drink and a food according to claim 4, wherein the presentation section superposes and displays the item evaluation value of the drink and the item evaluation value of the food on a radar chart of the plurality of evaluation items.

6. The apparatus for presenting information regarding compatibility between a drink and a food according to claim 4 or 5, wherein:

the specific correlation regarded as resulting in favorable compatibility is prepared in the form of a plurality of compatibility patterns respectively exhibiting different correlations, and

the presentation section presents information regarding a correlation between item evaluation values of a drink and a food determined to be a combination having favorable compatibility, with respect to each of the plurality of compatibility patterns.

7. An apparatus for presenting information regarding compatibility between a cuisine and a seasoning, the apparatus comprising:

a cuisine item evaluation value storage section in which an item evaluation value for each of a plurality of evaluation items for determination of compatibility with a seasoning in terms of one or a combination of two or more of taste, odor, and eating quality, in association with a cuisine item and a seasoning category of a pairing target, is stored as a relative numerical value with respect to an item reference value;

a seasoning item evaluation value storage section in which an item evaluation value for each of the plurality of evaluation items for determination of compatibility with a cuisine in terms of one or a combination of two or more of taste, odor, and eating quality, in association with a seasoning item and a cuisine category of a pairing target, is stored as a relative numerical value with respect to an item reference value;

a reception section that receives a request for presentation of information regarding a combination having favorable compatibility between a cuisine and a seasoning;

a determination section that extracts item evaluation values of cuisine and a seasoning in the combination according to the presentation request received by the reception section, from each of the cuisine item evaluation value storage section and the seasoning item evaluation value storage section, and, in a case in which a correlation between the extracted item evaluation values for the cuisine and the seasoning is matched to a specific correlation regarded as resulting in favorable compatibility, determines the cuisine and the seasoning to be a combination having favorable compatibility; and

a presentation section that presents information regarding the cuisine and the seasoning determined by the determination section to be the combination having favorable compatibility.

8. An apparatus for presenting information regarding compatibility between a cuisine and a seasoning, the apparatus comprising:

a cuisine item evaluation value storage section in which an item evaluation value for each of a plurality of evaluation items for determination of compatibility with a seasoning in terms of one or a combination of two or more of taste, odor, and eating quality, in association with a cuisine item and a seasoning category of a pairing target, is stored as a relative numerical value with respect to an item reference value;

a seasoning item evaluation value storage section in which an item evaluation value for each of the plurality of evaluation items for determination of compatibility with a cuisine in terms of one or a combination of two or more of taste, odor, and eating quality, in association with a seasoning item and a cuisine category of a pairing target, is stored as a relative numerical value with respect to an item reference value;

a cuisine-seasoning combination item evaluation value storage section in which item evaluation values of a cuisine and a seasoning in a specific combination, in which a correlation between item evaluation values of a cuisine and a seasoning stored in the cuisine item evaluation value storage section and the seasoning item evaluation value storage section is matched to a specific correlation regarded as resulting in favorable compatibility, are stored;

a reception section that receives a request for presentation of information regarding a combination having favorable compatibility between a cuisine and a seasoning; and

a presentation section that extracts item evaluation values for a cuisine and a seasoning in the combination according to the presentation request received by the reception section, from the cuisine-seasoning combination item evaluation value storage section, and presents information regarding the cuisine and the seasoning extracted.

9. An apparatus for presenting information regarding compatibility between seasonings, the apparatus comprising:

a seasoning item evaluation value storage section in which an item evaluation value for each of a plurality of evaluation items for determination of compatibility with another seasoning in terms of one or a combination of two or more of taste, odor, and eating quality, in association with items of seasonings, is stored as a relative numerical value with respect to an item reference value;

a reception section that receives a request for presentation of information regarding a combination having favorable compatibility between seasonings;

a determination section that extracts item evaluation values of respective seasonings in the combination according to the presentation request received by the reception section, from the seasoning item evaluation value storage section, and, in a case in which a correlation between the extracted item evaluation values for the seasonings is matched to a specific correlation regarded as resulting in favorable compatibility, determines the seasonings to be a combination having favorable compatibility; and

a presentation section that presents information regarding the seasonings determined by the determination section to be the combination having favorable compatibility.

10. An apparatus for presenting information regarding compatibility between seasonings, the apparatus comprising:

a seasoning item evaluation value storage section in which an item evaluation value for each of a plurality of evaluation items for determination of compatibility with another seasoning in terms of one or a combination of two or more of taste, odor, and eating quality, in association with items of seasonings, is stored as a relative numerical value with respect to an item reference value;

a seasoning combination item evaluation value storage section in which item evaluation values of respective seasonings in a specific combination, in which a correlation between respective item evaluation values of seasonings stored in the seasoning item evaluation value storage section is a specific correlation regarded as resulting in favorable compatibility, are stored;

a reception section that receives a request for presentation of information regarding a combination having favorable compatibility between seasonings; and

a presentation section that extracts item evaluation values for seasonings in the combination according to the presentation request received by the reception section, from the seasoning combination item evaluation value storage section, and presents information regarding the seasonings extracted.

11. An apparatus for presenting information regarding compatibility between food components, the apparatus comprising:

a food component evaluation value storage section in which an evaluation value for each of a plurality of evaluation items for determination of compatibility with another food component in terms of one or a combination of two or more of taste, odor, and eating quality, in association with food components constituting a food, is stored as a relative numerical value with respect to a food component reference value;

a reception section that receives a request for presentation of information regarding a combination having favorable compatibility between food components;

a determination section that extracts evaluation values of respective food components in the combination according to the presentation request received by the reception section, from the food component evaluation value storage section, and, in a case in which a correlation between the extracted evaluation values of food components is matched to a specific correlation regarded as resulting in favorable compatibility, determines the food components to be a combination having favorable compatibility; and

a presentation section that presents information regarding the food components determined by the determination section to be the combination having favorable compatibility.

12. An apparatus for presenting information regarding compatibility between food components, the apparatus comprising:

a food component evaluation value storage section in which an evaluation value for each of a plurality of evaluation items for determination of compatibility with another food component in terms of one or a combination of two or more of taste, odor, and eating quality, in association with food components, is stored as a relative numerical value with respect to a food component reference value;

a food component combination evaluation value storage section in which evaluation values of respective food components in a specific combination, in which a correlation between respective evaluation values of food components stored in the food component evaluation value storage section is matched to a specific correlation regarded as resulting in favorable compatibility, are stored;

a reception section that receives a request for presentation of information regarding a combination having favorable compatibility between food components; and

a presentation section that extracts respective item evaluation values for food components in the combination according to the presentation request received by the reception section, from the food component combination item evaluation value storage section, and presents information regarding the food components extracted.

13. The apparatus for presenting information regarding compatibility between food components according to claim 11 or 12, wherein the plurality of evaluation items comprises hardness, viscosity and elasticity of a food component.

14. A method of diagnosing compatibility between a drink and a food, comprising diagnosing compatibility between a drink and a food by use of a detection value in a taste sensor, wherein:

the taste sensor is configured to measure an evaluation value of an aftertaste of a food, in accordance with a detection value obtained by immersing a sensor section in a food sample liquid, then washing the sensor section with a reference liquid corresponding to human saliva, and then immersing the sensor section in the reference liquid,

an evaluation value of an aftertaste of a food obtained by rinsing with a drink is measured in accordance with a detection value obtained by washing the sensor section with a drink as a compatibility diagnosis target instead of washing the sensor section with the reference liquid, and then immersing the sensor section in the reference liquid, and

compatibility of the drink and the food is diagnosed depending on the evaluation value of the aftertaste of the food obtained by rinsing with the drink.

15. The apparatus for presenting information regarding compatibility between a drink and a food according to claim 1 or 4, further comprising a user history data storage section in which user-specific history data showing a food item previously purchased by or supplied to a user, in association with the user, is stored,

wherein, in a case in which the presentation request is received by the reception section, a food item is selected from the user-specific history data stored in the user history data storage section, in accordance with the presentation request, and a drink item having favorable compatibility with the selected food item is displayed on a display screen of a user terminal.

16. The apparatus for presenting information regarding compatibility between a drink and a food according to claim 1 or 4, further comprising a user history data storage section in which user-specific history data showing a drink item previously purchased by or supplied to a user, in association with the user, is stored,

wherein, in a case in which the presentation request is received by the reception section, a drink item is selected from the user-specific history data stored in the user history data storage section, in accordance with the presentation request, and a food item having favorable compatibility with the selected drink item is displayed on a display screen of a user terminal.

17. The apparatus for presenting information regarding compatibility between a cuisine and a seasoning according to claim 7 or 8, further comprising a user history data storage section in which user-specific history data showing a cuisine item previously purchased by or supplied to a user, in association with the user, is stored,

wherein, in a case in which the presentation request is received by the reception section, a cuisine item is selected from the user-specific history data stored in the user history data storage section, in accordance with the presentation request, and a seasoning item having favorable compatibility with the selected cuisine item is displayed on a display screen of a user terminal.

18. The apparatus for presenting information regarding compatibility between a cuisine and a seasoning according to claim 7 or 8, further comprising a user history data storage section in which user-specific history data showing a seasoning item previously purchased by or supplied to a user, in association with the user, is stored,
wherein, in a case in which the presentation request is received by the reception section, a seasoning item is selected from the user-specific history data stored in the user history data storage section, in accordance with the presentation request, and a cuisine item having favorable compatibility with the selected seasoning item is displayed on a display screen of a user terminal.

19. The apparatus for presenting information regarding compatibility between seasonings according to claim 9 or 10, further comprising a user history data storage section in which user-specific history data showing a seasoning item previously purchased by or supplied to a user, in association with the user, is stored,
wherein, in a case in which the presentation request is received by the reception section, a seasoning item is selected from the user-specific history data stored in the user history data storage section, in accordance with the presentation request, and another seasoning item having favorable compatibility with the selected seasoning item is displayed on a display screen of a user terminal.

20. The apparatus for presenting information regarding compatibility between food components according to claim 11 or 12, further comprising a user history data storage section in which user-specific history data showing a food component previously purchased by or supplied to a user, in association with the user, is stored,
wherein, in a case in which the presentation request is received by the reception section, a food component is selected from the user-specific history data stored in the user history data storage section, in accordance with the presentation request, and another food component having favorable compatibility with the selected food component is displayed on a display screen of a user terminal.

21. The apparatus for presenting information regarding compatibility between a drink and a food according to claim 16, wherein a food item providing a calorie intake in an acceptable range for the user is selected from the user-specific history data.

22. The apparatus for presenting information regarding compatibility between a cuisine and a seasoning according to claim 18, wherein a cuisine item providing a calorie intake in an acceptable range for the user is selected from the user-specific history data.

23. The apparatus for presenting information regarding compatibility between food components according to claim 20, wherein a food component item providing a calorie intake in an acceptable range for the user is selected from the user-specific history data.

FIG.1

100

200

300

120

FIG.2

100,120,200

```
                11                              16
        ┌──────────────┐              ┌──────────────┐
        │     CPU      │──────┬───────│    INPUT     │
        └──────────────┘      │       │  APPARATUS   │
                              │       └──────────────┘
                12            │                 17
        ┌──────────────┐      │       ┌──────────────┐
        │     ROM      │──────┼───────│   DISPLAY    │
        └──────────────┘      │       │  APPARATUS   │
                              │       └──────────────┘
                13            │                 18
        ┌──────────────┐      │       ┌──────────────┐
        │     RAM      │──────┼───────│ CONNECTION   │
        └──────────────┘      │       │     I/F      │
                              │       └──────────────┘
                              │ 
                             ~15
        ┌──────────────┐      │
        │   STORAGE    │──────┘
        └──────────────┘
```

# FIG.3

200,100,120

1030

RECEPTION
SECTION

1040

DETERMINATION
SECTION

1050

PRESENTATION
SECTION

1010

DRINK ITEM
EVALUATION VALUE
STORAGE SECTION

1020

FOOD ITEM
EVALUATION VALUE
STORAGE SECTION

1025

COMPATIBILITY
PATTERN
STORAGE SECTION

FIG.4A

FIG.4B

SULFUR-BASED

ESTER-BASED

AMINE-BASED

ISV

IV

ALDEHYDE
-BASED

ORGANIC
ACID-BASED

FIG.4C

# FIG.5A

「STRINGENCY-RICHNESS」

IV
ISV

⟺ CATEGORY:
"MEAT DISH"

「SOURNESS」   「DEEPNESS」

ITEM: "SPECIFIC BRAND OF WHITE WINE"

## FIG.5B

「ASTRINGENCY-RICHNESS」

IV
ISV

CATEGORY:
"WHITE WINE"

「SOURNESS」 「DEEPNESS」

ITEM: "GINGER PORK LOIN"

# FIG.6A

「BITTERNESS-RICHNESS」

IV
ISV

⟷ CATEGORY: "SNACK"

「SOURNESS-SHARPNESS」　　「SALTINESS-STRONGNESS」

ITEM: "SPECIFIC BRAND OF BEER"

FIG.6B

「BITTERNESS-RICHNESS」

ISV

IV

CATEGORY: "BEER"

「SOURNESS-SHARPNESS」 「SALTINESS-STRONGNESS」

ITEM: "ZHA CAI"

FIG.7

「1」

「2」 「3」
PT1

「1」

「2」 「3」
PT2

「1」

「2」 「3」
PT3

「1」

「2」 「3」
PT4

# FIG.8

# FIG.9

510

"SPECIFIC BRAND OF WHITE WINE" AND "GINGER PORK LOIN"
PROVIDE COMBINATION HAVING FAVORABLE COMPATIBILITY

「ASTRINGENCY-RICHNESS」

DRINK — FOOD

「SOURNESS」 「DEEPNESS」

"SPECIFIC BRAND OF WHITE WINE" AND "GINGER PORK LOIN"
EXHIBIT ITEM EVALUATION VALUES IV OF THREE EVALUATION
ITEMS WELL-BALANCED AND "TUNED"

# FIG.10

510

"SPECIFIC BRAND OF WHITE WINE" AND "GINGER PORK LOIN" PROVIDE COMBINATION HAVING FAVORABLE COMPATIBILITY

「ASTRINGENCY-RICHNESS」

DRINK — FOOD

「SOURNESS」 「DEEPNESS」

"SPECIFIC BRAND OF WHITE WINE" AND THE "GINGER PORK LOIN" EXHIBIT ITEM EVALUATION VALUES IV OF THREE EVALUATION ITEMS WELL-BALANCED AND "TUNED"

"SPECIFIC BRAND OF WHITE WINE" AND "SALAD CHICKEN" PROVIDE COMBINATION HAVING FAVORABLE COMPATIBILITY

「BITTERNESS-RICHNESS」

DRINK — FOOD

「SOURNESS -SHARPNESS」 「SALTINESS -STRONGNESS」

"SPECIFIC BRAND OF WHITE WINE" AND THE "SALAD CHICKEN" ARE COMPLEMENTED WITH EACH OTHER SO THAT ONE PROVIDES ANY TASTE NOT PROVIDED BY OTHER

# FIG.11

「1」

「2」        「3」

PT4

「ASTRINGENCY-
RICHNESS」

IV
ISV

「SOURNESS」        「DEEPNESS」

ITEM: "SPECIFIC BRAND
OF WHITE WINE"

⟷

「ASTRINGENCY-
RICHNESS」

ISV
IV

「SOURNESS」        「DEEPNESS」

ITEM: "SALAD CHICKEN"

FIG.12

510

"SPECIFIC BRAND OF BEER" AND "ZHA CAI" PROVIDE COMBINATION HAVING FAVORABLE COMPATIBILITY

「BITTERNESS-RICHNESS」

DRINK
FOOD

「SOURNESS-SHARPNESS」　「SALTINESS-STRONGNESS」

"SPECIFIC BRAND OF BEER" AND "ZHA CAI" PROVIDE SAVOR IN WHICH TASTE "SOURNESS-SHARPNESS"
NOT PROVIDED BY ONE IS COMPLEMENTED BY THAT PROVIDED BY OTHER AND "SOURNESS-SHARPNESS" STANDS OUT

"SPECIFIC BRAND OF BEER" AND "BOILED SOYBEAN"
PROVIDE COMBINATION HAVING FAVORABLE COMPATIBILITY

「BITTERNESS-RICHNESS」

FOOD
DRINK

「SOURNESS-
SHARPNESS」　「SALTINESS-
STRONGNESS」

"SPECIFIC BRAND OF BEER" AND "BOILED SOYBEAN"
PROVIDE SAVOR IN WHICH TASTE
"SALTINESS-STRONGNESS" NOT PROVIDED BY ONE IS
COMPLEMENTED BY THAT PROVIDED BY OTHER
AND "SALTINESS-STRONGNESS" STANDS OUT

"SPECIFIC BRAND OF BEER" AND "CHAR-BROILED CHICKEN"
PROVIDE COMBINATION HAVING FAVORABLE COMPATIBILITY

「BITTERNESS-RICHNESS」

DRINK
FOOD

「SOURNESS-
SHARPNESS」　「SALTINESS-
STRONGNESS」

"SPECIFIC BRAND OF BEER" AND
"CHAR-BROILED CHICKEN" PROVIDE SAVOR IN WHICH
TASTE "BITTERNESS-RICHNESS" NOT PROVIDED BY
ONE IS COMPLEMENTED BY THAT PROVIDED BY
OTHER AND "BITTERNESS-RICHNESS" STANDS OUT

EP 4 170 577 A1

## FIG.13A

ITEM: "SPECIFIC BRAND OF BEER"          ITEM: "ZHA CAI"

FIG.13B

ITEM: "SPECIFIC BRAND OF BEER"    ITEM: "BOILED SOYBEAN"

## FIG.13C

ITEM: "SPECIFIC BRAND OF BEER"    ITEM: "CHAR-BROILED CHICKEN"

# FIG.14

200,100,120

1030
RECEPTION
SECTION

1010
DRINK ITEM
EVALUATION VALUE
STORAGE SECTION

1020
FOOD ITEM
EVALUATION VALUE
STORAGE SECTION

1050
PRESENTATION
SECTION

1025
COMPATIBILITY
PATTERN
STORAGE SECTION

2010
DRINK-FOOD
COMBINATION ITEM
EVALUATION VALUE
STORAGE SECTION

# FIG.15

510

"ROAST FISH (SALT-GRILLED PACIFIC SAURY)" AND "CITRUS JUICE (CITRUS SUDACHI JUICE)" PROVIDE COMBINATION HAVING FAVORABLE COMPATIBILITY

「BITTERNESS-RICHNESS」

CITRUS JUICE (CITRUS SUDACHI JUICE)

「SOURNESS」  「DEEPNESS」

ROAST FISH (SALT-GRILLED PACIFIC SAURY)

COMBINATION OF "ROAST FISH (SALT-GRILLED PACIFIC SAURY)" AND "CITRUS JUICE (CITRUS SUDACHI JUICE)" CORRESPONDS TO PATTERN IN WHICH TASTE "SOURNESS" NOT PROVIDED BY ONE "ROAST FISH (SALT-GRILLED PACIFIC SAURY)" IS COMPLEMENTED BY THAT PROVIDED BY OTHER "CITRUS JUICE (CITRUS SUDACHI JUICE)" AND "SOURNESS" IS ALLOWED TO STAND OUT. TEXTURE HIGH IN SATISFACTION LEVEL IS ACHIEVED WITH SOURNESS EVEN BY NO ADDITIONAL TASTING WITH SALTINESS. SALT REDUCTION EFFECT IS EXERTED.

# FIG.16

510

"DEEP-FRIED FOOD" AND "LEMON JUICE" PROVIDE
COMBINATION HAVING FAVORABLE COMPATIBILITY

「BITTERNESS-RICHNESS」

DEEP-FRIED FOOD

LEMON JUICE

「SOURNESS」　　　　　　　　　　　　　　　　「DEEPNESS」

COMBINATION OF "DEEP-FRIED FOOD" AND THE "LEMON JUICE"
CORRESPONDS TO PATTERN IN WHICH "SOURNESS" OF SEASONING
AND "DEEPNESS" OF DEEP-FRIED FOOD ARE COMPLEMENTED WITH
EACH OTHER AND SUCH TASTES ARE HARMONIZED.
SALT REDUCTION EFFECT IS EXPECTED DEPENDING
ON COMBINATION OF CUISINE AND SEASONING.

# FIG.17

510

"BROTH SOY SAUCE" AND "CITRUS FRUIT" PROVIDE
COMBINATION HAVING FAVORABLE COMPATIBILITY

「BITTERNESS-RICHNESS」

BROTH SOY SAUCE

CITRUS FRUIT

「SOURNESS」                    「DEEPNESS」

COMBINATION OF "BROTH SOY SAUCE" AND THE "CITRUS FRUIT"
CORRESPONDS TO PATTERN IN WHICH "SOURNESS" OF CITRUS FRUIT
AND "DEEPNESS" OF BROTH SOY SAUCE ARE COMPLEMENTED WITH
EACH OTHER AND SUCH TASTES ARE HARMONIZED.
COOKING EXAMPLE OF COMBINATION OF BROTH SOY SAUCE
AND CITRUS FRUIT IS PONZU SAUCE

# FIG.18

510

"SOUP STOCK (DRIED BONITO/DRIED SEA SLUG)"
AND "BEAN PASTE" PROVIDE COMBINATION HAVING
FAVORABLE COMPATIBILITY

「BITTERNESS-RICHNESS」

SOUP STOCK (DRIED BONITO/
DRIED SEA SLUG)

BEAN PASTE

「SOURNESS」          「DEEPNESS」

COMBINATION OF "SOUP STOCK (DRIED BONITO/DRIED SEA SLUG)" AND
"BEAN PASTE" CORRESPONDS TO PATTERN IN WHICH ITEM EVALUATION
VALUES IV OF THREE EVALUATION ITEMS ARE APPROXIMATED AND
THUS TASTES ARE SYNERGISTICALLY DEEPENED. COOKING EXAMPLE
OF COMBINATION OF SOUP STOCK (DRIED BONITO/DRIED SEA SLUG)
AND BEAN PASTE IS BEAN PASTE SOUP.

# FIG.19

510

"PORK BONE CHINESE NOODLE SOUP" AND "BEAN PASTE"
PROVIDE COMBINATION HAVING FAVORABLE COMPATIBILITY

「BITTERNESS–RICHNESS」

PORK BONE CHINESE
NOODLE SOUP

BEAN PASTE

「SOURNESS」    「DEEPNESS」

COMBINATION OF "PORK BONE CHINESE NOODLE SOUP" AND THE "BEAN
PASTE" CORRESPONDS TO PATTERN IN WHICH TASTE "DEEPNESS" NOT
PROVIDED BY ONE "PORK BONE CHINESE NOODLE SOUP" IS
COMPLEMENTED BY THAT PROVIDED BY OTHER "BEAN PASTE" AND
"DEEPNESS" IS ALLOWED TO STAND OUT. COOKING EXAMPLE OF
COMBINATION OF PORK BONE CHINESE NOODLE SOUP AND BEAN PASTE
IS PORK BONE BEAN PASTE CHINESE NOODLE SOUP.

# FIG.20A

HARDNESS

IV

VISCOSITY

———— ONE FOOD COMPONENT

—·——·— OTHER FOOD COMPONENT

ELASTICITY

CONTRASTED COMPATIBILITY PATTERN PT11

## FIG.20B

TUNED COMPATIBILITY PATTERN PT12

FIG.20C

MIXED COMPATIBILITY PATTERN PT13

## FIG.21

510

"FRIED EGG" AND "CRISPY BACON" PROVIDE
COMBINATION HAVING FAVORABLE COMPATIBILITY.

HARDNESS

CRISPY BACON

FRIED EGG

VISCOSITY

ELASTICITY

COMBINATION OF "FRIED EGG" AND "CRISPY BACON" CORRESPONDS
TO PATTERN IN WHICH OTHER FOOD COMPONENT HAS TEXTURE NOT
PROVIDED BY ONE FOOD COMPONENT AND CONTRASTIVE TEXTURES
(SOFT/HARD) ARE OBTAINED.
COOKING EXAMPLE: BACON AND EGGS

# FIG.22

510

"FERMENTED SOYBEAN" AND "OKRA" PROVIDE
COMBINATION HAVING FAVORABLE COMPATIBILITY

HARDNESS

FERMENTED
SOYBEAN

VISCOSITY

OKRA

ELASTICITY

COMBINATION OF "FERMENTED SOYBEAN" AND "OKRA" CORRESPONDS
TO PATTERN IN WHICH TEXTURES ARE RESEMBLANT TO EACH OTHER
AND TUNED TEXTURE (STRONGLY STICKING TEXTURE) IS OBTAINED.
COOKING EXAMPLE: OKRA/FERMENTED SOYBEAN

# FIG.23

# FIG.24

| NAMES | ODOR QUALITY |
|---|---|
| 2-BUTYL-2-OCTENAL | MEAT |
| 1,2-DIMETHYLBENZENE (= O-XYLENE) | GERANIUM |
| ACETOALDEHYDE | ETHER, FLOWER, FRUIT, GREEN APPLE, SWEET |
| 2-METHOXY-4-VINYLPHENOL (-4-VINYLGUAIACOL) | CLOVE, CURRY, SMOKE, SPICE, WOOD |
| DIMETHYL SULFIDE (= THIOBISMETHANE, METHYLTHIOMETHANE, METHYL SULFIDE) | CABBAGE, GASOLINE, ORGANIC, SULFUR, WET SOIL |
| BENZOTHIAZOLE (= 1,3-BENZOTHIAZOLE) | GASOLINE, LEATHER, DRUG, NUT, RUBBER |
| 2-BUTANONE (= METHYL ETHYL KETONE) | ETHER, SPICY, FRUIT, PLEASING, SWEET |
| MALTOL | CARAMEL, CANDYFLOSS, MALT, ROAST BREAD, ROAST NUT |
| 3-ETHYL-2,5-DIMETHYLPYRAZINE | SOUP, SOIL, POTATO, ROAST |
| 2,6,6-TRIMETHYL-1-CYCLOHEXEN-1-YL)-3-BUTEN-2-ONE | FLORAL, SWEET, VIOLET |
| 2-BUTYL-1-OCTANOL | FLOWER, AROMA |
| DODECANAL | CITRUS FRUIT, FAT, LILY |
| ETHYL HEXADECANOATE (-ETHYL HEXADECANOATE) | WAX |
| Y-MUUROLENE | HERB, SPICE, WOOD |
| PENTANAL (= VALERALDEHYDE) | ALMOND, GREEN, MALT, OIL, IRRITATION, BURNING SWEET-SOUR AND SMOKY ODOR |
| 2-METHYLPROPANAL (ISOBUTANOL, ISOBUTYLALDEHYDE) | CARAMEL, COCOA, GREEN, MALT, NUT, SWEET-SOUR AND SMOKY IRRITATING ODOR |

EP 4 170 577 A1

# FIG.25

| SAKE | 7 |
|---|---|
| RED WINE | 34 |
| WHITE WINE | 39 |
| BEER | 39 |
| COGNAC | 38 |
| GIN | 23 |

FIG.26

FIG.27

# FIG.28A

STEP1

Vr DETECTION

STEP2

Vs DETECTION
Vs−Vr=FIRST TASTE
MEASUREMENT VALUE

STEP3

STEP4

Vr' DETECTION
Vr'−Vr=AFTERTASTE
MEASUREMENT VALUE

STEP5

# FIG.28B

STEP10

10

11

RS

Vr DETECTION

STEP20

10

11

SI

Vs DETECTION
Vs−Vr=FIRST TASTE
MEASUREMENT VALUE

STEP30

10

11

TSI

STEP40

10

11

RS

Vtr DETECTION
Vtr−Vr=FLUSHING
AFTERTASTE
MEASUREMENT VALUE

STEP50

10

11

AL

FIG.29A

## FIG.29B

# FIG.30A

# FIG.30B

FIG.31

"SPECIFIC BRAND IT6 OF SAKE" HAS FLUSHING EFFECT LESS THAN THAT OF COKE, AND IS EXCELLENT IN CONTINUOUS EATING PROPERTIES

COMBINATION OF "PIZZA (MARGHERITA)" AND "SPECIFIC BRAND IT6 OF SAKE" CORRESPONDS TO PATTERN IN WHICH "SHARPNESS" OF PIZZA (MARGHERITA) AND "DEEPNESS" OF BRAND IT6 OF SAKE ARE COMPLEMENTED WITH EACH OTHER AND SUCH TASTES ARE HARMONIZED.

EP 4 170 577 A1

## FIG.32

510

COMBINATION OF "JJIGAE SOUP" AND "SPECIFIC BRAND IT7 OF SAKE (UNREFINED SAKE)" CORRESPONDS TO PATTERN IN WHICH "SOURNESS-SHARPNESS" OF JJIGAE AND "AFTERGLOW OF UMAMI" OF SPECIFIC BRAND IT7 OF SAKE (UNREFINED SAKE) ARE COMPLEMENTED WITH EACH OTHER AND SUCH TASTES ARE HARMONIZED.

COMBINATION OF "JJIGAE SOUP" AND "SPECIFIC BRAND IT7 OF SAKE (UNREFINED SAKE)" CORRESPONDS TO PATTERN IN WHICH DEEPNESS IS COMPLEMENTED BY SPECIFIC BRAND IT7 OF SAKE (UNREFINED SAKE).

EP 4 170 577 A1

# FIG.33

200,100,120

1030
RECEPTION SECTION

1040
DETERMINATION SECTION

1050
PRESENTATION SECTION

1010
DRINK ITEM EVALUATION VALUE STORAGE SECTION

1020
FOOD ITEM EVALUATION VALUE STORAGE SECTION

1060
BUYING HISTORY DATA STORAGE SECTION

1025
COMPATIBILITY PATTERN STORAGE SECTION

# FIG.34

200,100,120

1030
RECEPTION
SECTION

1010
DRINK ITEM
EVALUATION VALUE
STORAGE SECTION

1050
PRESENTATION
SECTION

1020
FOOD ITEM
EVALUATION VALUE
STORAGE SECTION

1025
COMPATIBILITY
PATTERN
STORAGE SECTION

1060
BUYING HISTORY
DATA STORAGE
SECTION

2010
DRINK-FOOD
COMBINATION ITEM
EVALUATION VALUE
STORAGE SECTION

# FIG.35A

531

TASTE CHART OF IT11
WHICH YOU ALWAYS BUY
BEER WHICH YOU CAN ENJOY LUSH
RICHNESS AND GENEROUS FRAGRANCE

530A

TODAY'S RECOMMENDED PREPARED FOOD
FOR YOU IS ITEM IT21 "FRIED BURDOCK ROOT"

TODAY'S RECOMMENDED PREPARED FOOD
FOR YOU IS ITEM IT22 "SIMMERED HIJIKI"

TODAY'S RECOMMENDED PREPARED FOOD FOR
YOU IS ITEM IT23 "CHAR-BROILED CHICKEN"

# FIG.35B

531

TASTE CHART OF IT
WHICH YOU ALWAYS BUY
BEER WHICH YOU CAN ENJOY LUSH
RICHNESS AND GENEROUS FRAGRANCE

【BITTERNESS】IT
8.0
6.0
4.0
2.0
0.0
2.0
4.0
6.0
8.0

【SHARPNESS】　　　　　　　【UMAMI】

【AFTERGLOW
OF UMAMI】　　　　　　　　【DENSENESS】

【AFTERTASTE OF BITTERNESS】

530B

TODAY'S RECOMMENDED PREPARED FOOD FOR YOU IS ITEM IT21
"FRIED BURDOCK ROOT" IN CONSIDERATION OF YOUR ACCEPTABLE
CALORIE INTAKE

IT21　BITTERNESS　IV
ISV
SOURNESS　　　　　　UMAMI

SWEETNESS　SALTINESS

## INTERNATIONAL SEARCH REPORT

| International application No. |
| --- |
| **PCT/JP2022/019734** |

**A. CLASSIFICATION OF SUBJECT MATTER**

*G06Q 50/10*(2012.01)i; *G06Q 30/02*(2012.01)i
FI: G06Q50/10; G06Q30/02 470

According to International Patent Classification (IPC) or to both national classification and IPC

**B. FIELDS SEARCHED**

Minimum documentation searched (classification system followed by classification symbols)

G06Q10/00-99/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2016-9211 A (TOA SHOJI CO., LTD.) 18 January 2016 (2016-01-18) paragraphs [0018]-[0022], [0025]-[0030], [0033] | 1-23 |
| A | JP 2017-130142 A (UCC UESHIMA COFFEE CO., LTD.) 27 July 2017 (2017-07-27) paragraphs [0026], [0029], [0032], [0043], [0049]-[0054] | 1-23 |
| A | JP 2013-89006 A (SEIKO EPSON CORP.) 13 May 2013 (2013-05-13) paragraphs [0027]-[0030], [0037]-[0040], [0053]-[0061] | 1-23 |
| A | JP 2020-140242 A (YAHOO JAPAN CORP.) 03 September 2020 (2020-09-03) paragraphs [0010]-[0015], [0022]-[0040], [0055]-[0066] | 1-23 |
| A | JP 2017-204194 A (NIPPON HOSO KYOKAI) 16 November 2017 (2017-11-16) paragraphs [0014]-[0023] | 1-23 |

☐ Further documents are listed in the continuation of Box C.      ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **28 July 2022** | **09 August 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2022/019734** |

| Patent document cited in search report | | | Publication date (day/month/year) | Patent family member(s) | Publication date (day/month/year) |
| --- | --- | --- | --- | --- | --- |
| JP | 2016-9211 | A | 18 January 2016 | (Family: none) | |
| JP | 2017-130142 | A | 27 July 2017 | (Family: none) | |
| JP | 2013-89006 | A | 13 May 2013 | (Family: none) | |
| JP | 2020-140242 | A | 03 September 2020 | (Family: none) | |
| JP | 2017-204194 | A | 16 November 2017 | (Family: none) | |

Form PCT/ISA/210 (patent family annex) (January 2015)

**EP 4 170 577 A1**

REFERENCES CITED IN THE DESCRIPTION

**Patent documents cited in the description**

- JP 2017130142 A
- JP 2021081158 A